(19)

Europäisches
Patentamt
European
Patent Office
Office européen
des brevets

(11) **EP 4 563 982 A1**

(12) **EUROPEAN PATENT APPLICATION**

(43) Date of publication:
**04.06.2025 Bulletin 2025/23**

(21) Application number: **24216343.4**

(22) Date of filing: **29.11.2024**

(51) International Patent Classification (IPC):
*G01N 21/64* $^{(2006.01)}$  *C12Q 1/6869* $^{(2018.01)}$

(52) Cooperative Patent Classification (CPC):
(C-Sets available)
**G01N 21/6456; C12Q 1/6869;** G01N 2021/6419;
G01N 2021/6421; G01N 2021/6478 (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **01.12.2023 CN 202311657526**
**01.12.2023 CN 202323276402 U**
**01.12.2023 CN 202323288757 U**

(71) Applicant: **GeneMind Biosciences Company
Limited
Shenzhen City, 518023 Guangdong (CN)**

(72) Inventors:
• **XU, Jiwen**
**Shenzhen City, 518023 (CN)**
• **LI, Yang**
**Shenzhen City, 518023 (CN)**
• **LI, Mingze**
**Shenzhen City, 518023 (CN)**
• **LUO, Qian**
**Shenzhen City, 518023 (CN)**
• **XIAO, Chenguang**
**Shenzhen City, 518023 (CN)**
• **XU, Jianfeng**
**Shenzhen City, 518023 (CN)**

(74) Representative: **Schiweck Weinzierl Koch
Patentanwälte Partnerschaft mbB
Ganghoferstraße 68 B
80339 München (DE)**

(54) **OPTOMECHANICAL SYSTEM AND SEQUENCING SYSTEM**

(57) The present disclosure relates to the field of optical device technology, and in particular, to an optomechanical system (10) and a sequencing system using the optomechanical system (10). The optomechanical system (10) comprises an illumination device (300), an imaging device (400), and a movable platform device (200). The illumination device (300) is configured for emitting an excitation light to irradiate a sample of interest (20) so as to excite the sample of interest (20) to generate an emission light. The imaging device (400) is configured for acquiring the emission light generated by the sample of interest (20) and forming an image. The movable platform device (200) is configured for driving the sample of interest (20) to translate and/or rotate relative to the imaging device (400), so as to enable the imaging device (400) to consecutively acquire the emission light generated by the sample of interest (20). According to the optomechanical system (10) in the embodiments of the present disclosure, by arranging the movable platform device (200), the sample of interest (20) can be driven to move and/or rotate relative to the imaging device (400) during the sequencing process, such that the imaging device (400) can consecutively acquire the emission light generated by the sample of interest (20). As such, the platform moving time in the sequencing photographing process and the image acquisition time of the imaging device (400) are reduced, and a high-speed, high-throughput sequencing can thus be achieved.

FIG. 1

(52) Cooperative Patent Classification (CPC): (Cont.)

C-Sets
**C12Q 1/6869, C12Q 2535/122, C12Q 2565/518,
C12Q 2563/107**

**Description**

TECHNICAL FIELD

The present disclosure relates to the field of optical device technology, and in particular, to an optomechanical system and a sequencing system.

BACKGROUND

In gene sequencing devices in the prior art, area scan cameras are commonly used to acquire fluorescence signals generated by a biological sample and give an image output, and then biological information of the biological sample is acquired on the basis of the image. In the photographing process of the area scan camera, the movement of the biological sample and the exposure and imaging operations of the camera are conducted alternately, resulting in a time-consuming procedure. In sequencing devices using area scan exposure mode, the increase of sequencing throughput is generally achieved by improving the laser power. However, excessive power may easily lead to fluorescence quenching and thus failure of sequencing.

How to achieve high-speed, high-throughput sequencing is an important issue requiring urgent resolution in the art at present.

SUMMARY

The present disclosure provides an optomechanical system and a sequencing system that can achieve high-speed, high-throughput sequencing functionality.

In one aspect, the present disclosure provides an optomechanical system, including:

an illumination device, configured for emitting an excitation light to irradiate a sample of interest so as to excite the sample of interest to generate an emission light;
an imaging device, configured for acquiring the emission light and forming an image; and
a movable platform device, configured for driving the sample of interest to translate and/or rotate relative to the imaging device, so as to enable the imaging device to consecutively acquire the emission light.

According to the present disclosure, by arranging the movable platform device, the sample of interest can be driven to move and/or rotate relative to the imaging device during the sequencing process, such that the imaging device can consecutively acquire the emission light generated by the sample of interest. As such, the platform moving time in the sequencing photographing process and the image acquisition time of the imaging device are reduced, and a high-speed, high-throughput sequencing can thus be achieved.

In another aspect, the present disclosure further provides a sequencing system including the optomechanical system. Additional aspects and advantages of the present disclosure will in part be illustrated in the following description and become apparent from the following description, or may be learned by the implementation of the present disclosure.

BRIEF DESCRIPTION OF THE DRAWINGS

To more clearly illustrate the technical solutions in the embodiments of the present disclosure or the prior art, the drawings required for use in the description of the embodiments or the prior art will be briefly described below. It is obvious that the drawings in the description below are only some embodiments of the present disclosure, and other drawings can be derived from these drawings by those of ordinary skill in the art without creative efforts.

Among the drawings:

FIG. 1 is a perspective view of an optomechanical system according to an embodiment of the present disclosure;
FIG. 2 is a partial structural schematic of an optomechanical system according to an embodiment of the present disclosure;
FIG. 3 is a schematic top view of an imaging device according to an embodiment of the present disclosure;
FIG. 4 is a structural schematic of a combination of an illumination device, an imaging device, and an automatic focusing device according to an embodiment of the present disclosure;
FIG. 5 is a structural schematic of an optical path turning assembly according to an embodiment of the present disclosure;
FIG. 6 is a structural schematic of an automatic focusing device according to an embodiment of the present disclosure;
FIG. 7 is a partial exploded view of a lens assembly according to an embodiment of the present disclosure;
FIG. 8 is a schematic view of a combination of a bracket device and a movable platform device according to an embodiment of the present disclosure;
FIG. 9 is a partial enlarged view of A in FIG. 8;
FIG. 10 is a perspective view of a movable carrier platform according to an embodiment of the present disclosure;
FIG. 11 is an exploded view of a carrier structure according to an embodiment of the present disclosure;
FIG. 12 is a schematic view of a light-transmitting region of an objective lens according to an embodiment of the present disclosure;
FIG. 13 is a structural schematic of an illumination device and an imaging device according to the present disclosure;

FIG. 14 is a structural schematic of a shaping lens set according to the present disclosure;

FIG. 15 is a structural schematic of a camera assembly according to an embodiment of the present disclosure;

FIG. 16 is a spot diagram of a tube lens according to an embodiment of the present disclosure.

DETAILED DESCRIPTION

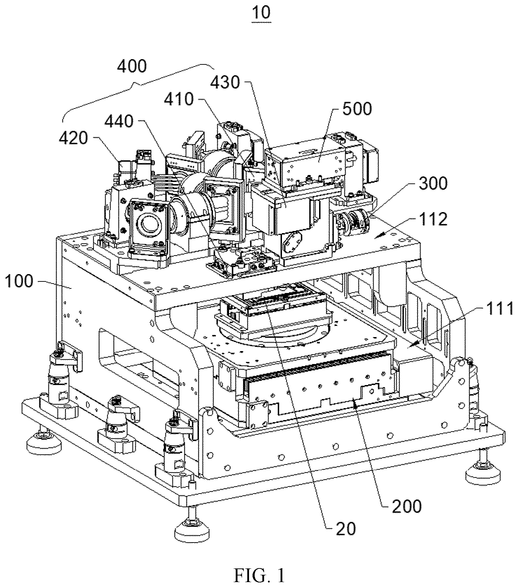

To make the objectives, technical solutions, and advantages of the present disclosure clearer, the technical solutions of the present disclosure are described below clearly and comprehensively in conjunction with the drawings in the present disclosure. It is apparent that the described embodiments are part of the embodiments of the present disclosure, but not all of them. Based on the embodiments of the present disclosure, all other embodiments obtained by those of ordinary skill in the art without creative efforts shall fall within the claimed scope of the present disclosure. Referring to FIGs. 1 to 11, an embodiment of the present disclosure provides an optomechanical system 10, including a bracket device 100, a movable platform device 200, an illumination device 300, and an imaging device 400; a cavity 111 is arranged in the bracket device 100, and a mounting surface 112 is arranged at the top of the bracket device 100; the movable platform device 200 is connected to the bracket device 100, and configured for carrying the sample of interest 20 and driving the sample of interest 20 to move in or out of the cavity 111; the sample of interest 20 is, for example, a flow cell with a nucleic acid molecule of interest immobilized on the surface. Specifically, the flow cell is provided with a fluid channel, the inner surface of the fluid channel is connected to a probe (such as an oligonucleotide) through chemical modification, and the nucleic acid molecule of interest is bonded to the probe via, e.g., hybridization and fixed on the flow cell. When bases with an optically detectable label (such as a fluorophore), polymerases, and the like are added into a fluid channel, the added bases are bonded to the nucleic acid molecule of interest according to the base complementary pairing principle. The optically detectable label is irradiated with the excitation light emitted by the illumination device 300 to generate emission light. The emission light may be, e.g., fluorescence generated by exciting the fluorophore with the excitation light. The emission light signals are acquired by the imaging device 400 to give an image, and finally, base calling is conducted according to the image, thereby achieving the base sequence determination of the nucleic acid molecule of interest.

As used herein, the "linear scanning" refers to a process where the movable platform device 100 drives the sample of interest 20 to move at a constant speed, and the imaging device 400 scans and images the sample of interest 20 using a time delay integration line scan camera (abbreviated as TDI camera). In the scanning process, the scanning speed of the imaging device 400 matches the moving speed of the movable platform device 100. The scanning speed of the imaging device 400 is dependent on the working horizontal scanning frequency of the TDI camera and the projection size of the pixels on the sample of interest 20 and is equal to the product of the two. The working horizontal scanning frequency of the TDI camera is the number of exposures of the light-sensing unit of the camera per second, and the projection size of the pixels of the TDI camera is dependent on the whole optical imaging system to meet the resolution and imaging requirement of the sample of interest 20.

The illumination device 300 is arranged on the mounting surface 112 and includes a light source configured for emitting an excitation light to irradiate the sample of interest 20 so as to excite the sample of interest 20 to generate an emission light. Specifically, for example, four bases A, T, C, and G bonded to the nucleic acid molecules of interest on the sample of interest 20 are labeled by different fluorophores. The light source is configured for emitting the excitation light with different wavelengths to irradiate the sample of interest 20, so as to excite the fluorophores to generate four different fluorescence signals.

The light source may be a multimode laser, and the excitation light emitted by the laser can form an illumination light with uniform intensity distribution after being homogenized by a multimode fiber, so as to avoid the influence on the sequencing results due to the brightness variation in the emission light signals after the excitation of the fluorophores and the amplification of the brightness variation after integral imaging.

In one embodiment, the output end face of the multimode fiber is rectangular, such that the spot of the excitation light output by the light source is rectangular.

In one embodiment, the illumination device 300 includes one or more light sources configured for emitting a plurality of excitation lights with different wavelengths to excite the sample of interest 20 to generate emission light signals with different wavelengths. In the scanning process of the TDI camera, the excitation light is generally provided with a high power to rapidly excite the sample of interest 20 to generate the emission light, so as to achieve fast scanning. If the plurality of excitation lights with different wavelengths simultaneously irradiate the same region of the sample of interest 20, the emission light signals generated by the sample of interest 20 may easily be quenched.

In order to avoid the quenching of the emission light signals generated by the sample of interest 20 due to the excessive power of the excitation lights when the plurality of excitation lights with different wavelengths simultaneously irradiate the same region of the sample of interest 20, it was found in studies that this disadvantageous influence can be eliminated by the excitation lights with different wavelengths emitted by the one or more light sources synchronously and respectively irradiating different regions of the sample of interest 20.

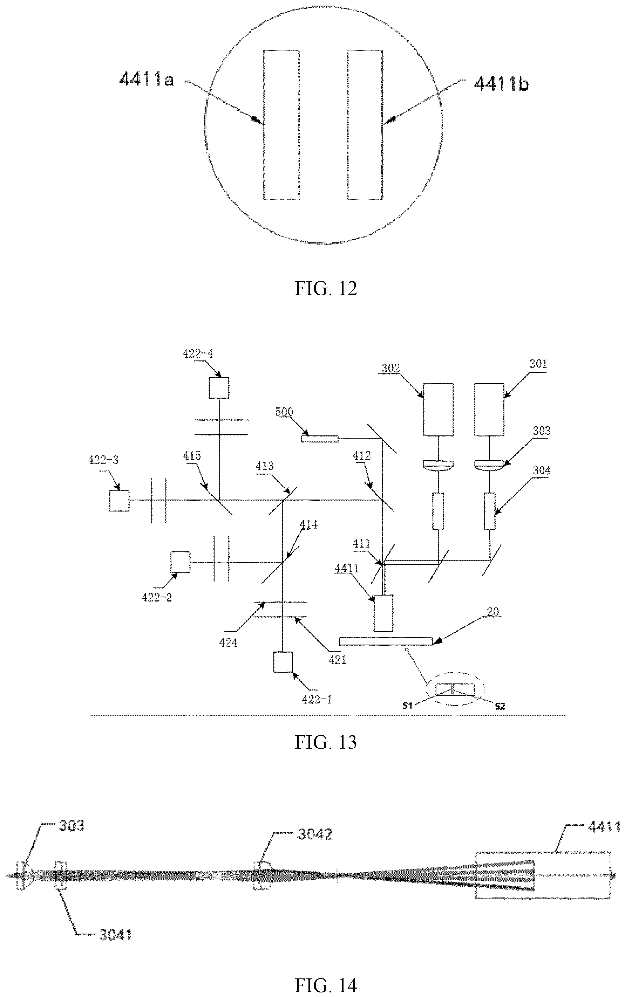

In one possible implementation, the plurality of excitation lights with different wavelengths respectively irradiate different regions of the sample of interest 20 along different illumination optical paths. Specifically, as shown in FIG. 13, the illumination device 300 includes a first light source 301 and a second light source 302. The first light source 301 emits an excitation light with a first wavelength to irradiate a first region of the sample of interest 20 along a first illumination optical path to form a first spot S1, so as to excite two types of bases bonded to the nucleic acid molecules of interest (for example, bases A and T) on the sample of interest 20 to generate different emission light signals; the second light source 302 emits an excitation light with a second wavelength to irradiate a second region of the sample of interest 20 along a second illumination optical path to form a second spot S2, so as to excite the other two types of bases bonded to the nucleic acid molecules of interest (for example, bases C and G) on the sample of interest 20 to generate different emission light signals, where the first spot and the second spot do not overlap. That is, the two excitation lights with different wavelengths emitted by the illumination device 300 irradiate different regions of the sample of interest 20 through different illumination optical paths, thus avoiding the quenching of the emission light generated by the sample of interest 20 due to the excessive power of the excitation lights when the two excitation lights with different wavelengths simultaneously irradiate the same region, and improving the sequencing quality. In one embodiment, the illumination device 300 may also include only one light source, and the light source can emit two excitation lights with different wavelengths simultaneously to irradiate the first region and the second region of the sample of interest 20 along different illumination optical paths, respectively, so as to increase the excitation efficiency and further increase the sequencing speed.

In one embodiment, as shown in FIG. 13, the illumination device 300 further includes a shaping lens set 304. The shaping lens set 304 is positioned on the illumination optical paths of the excitation lights, and configured for shaping the excitation light in a first direction and a second direction perpendicular to the first direction to adapt a shape and/or size of a spot of the excitation light to an imaging region of the imaging device 400, thus meeting the requirement of the imaging device 400 for performing a linear scanning on the sample of interest 20. The imaging region of the imaging device 400 is a light-sensing region of the TDI camera 422 in the imaging device 400. Specifically, the shaping lens set 304 includes a first shaping lens set and a second shaping lens set. The first shaping lens set is arranged on the first illumination optical path, and the excitation light with the first wavelength irradiates the first region of the sample of interest 20 after being shaped by the first shaping lens set to form an elongated first spot; the second shaping lens set is arranged on the second illumination optical path, and the excitation light emitted by the second light source irradiates the second region of the sample of interest 20 after being shaped by the second shaping lens set to form an elongated second spot. The elongated spots are rectangular spots with a high aspect ratio. In one embodiment, as shown in FIG. 13, the illumination device 300 further includes a collimation lens 303. The collimation lens 303 is configured for collimating the excitation lights emitted by the light sources. The collimation lens 303 may be positioned on an upstream or downstream optical path of the shaping lens set 304. Specifically, the collimation lens 303 includes a first collimation lens and a second collimation lens. The first collimation lens is positioned on an upstream or downstream optical path of the first shaping lens set, and the second collimation lens is positioned on an upstream or downstream optical path of the second shaping lens set.

In one embodiment, as shown in FIG. 14, the first shaping lens set and the second shaping lens set each include a first cylindrical lens 3041 and a second cylindrical lens 3042 sequentially arranged along the illumination optical path. The first cylindrical lens 3041 is configured for zooming the spot size or the beam angle of the excitation light in the first direction, and the second cylindrical lens 3042 is configured for zooming the spot size or the beam angle of the excitation light in the second direction, where the first direction is perpendicular to the second direction. Therefore, the excitation light with the first wavelength emitted by the first light source and the second excitation light emitted by the second light source can form an elongated spot after passing through the shaping lens set 304, and the shape and/or size of the elongated spot is adapted to the imaging region of the imaging device 400, thus meeting the requirement of the imaging device 400 for linear scanning.

As shown in FIG. 1, the imaging device 400 is arranged on the mounting surface 112, and configured for collecting the emission light signals generated by the sample of interest 20 after being excited by the excitation lights and for forming an image.

In the optomechanical system 10 of this embodiment, by setting the movable platform device 200 to cooperate with the sample of interest 20, the sample of interest 20 can be driven to move at a constant speed relative to the imaging device 400 during the sequencing process, such that the imaging device 400 can consecutively expose and scan different regions in the same line on the sample of interest 20 to acquire the emission light signals of the line; or the imaging device 400 transfers to the next line of the sample of interest 20 and consecutively exposes and scans different regions of the next line to acquire the emission light signals of that line. When the movable platform device 200 drives the sample of interest 20 to move at a constant speed, the scanning speed of the imaging device 400 matches the moving speed of the movable platform device 200, or otherwise, image information cannot be correctly formed.

Further, the movable platform device 200 can drive the sample of interest 20 to move at a constant speed, such that the imaging device 400 consecutively exposes and scans different regions of each line of the sample of interest 20 to acquire the emission light signals of the line, and the movable platform device 200 only accelerates or decelerates once when the imaging device 400 transfers to scan the next line of the sample of interest 20. Compared with the prior art where the movable platform device 200 accelerates and decelerates in every field of view in every line of the sample of interest 20 and the imaging device 400 exposes and acquires the emission light signals after the movable platform device 200 stops (that is, the movement and exposure/imaging of the sample of interest are conducted alternately), the movable platform device 200 in the optomechanical system 10 of the embodiment keeps linear movement at a high, constant speed, which greatly reduces the movement time of the movable platform device 200, allows consecutive exposure of the sample of interest 20 and high-bandwidth transmission of images during the movement, and requires no extra waiting time. As such, the time for photographing during the sequencing merely depends on the movement time of the movable platform device 200, thus greatly reducing the movement time of the movable platform device 200 and the image acquisition time of the imaging device 400 in the photographing process and improving the signal acquisition efficiency and the sequencing throughput. After the sequencing is completed, the sample of interest 20 is moved out by the movable platform device 200 to facilitate the removal and loading of the sample of interest 20, featuring convenience of use.

Meanwhile, the illumination device 300 emits two excitation lights with different wavelengths to the sample of interest 20 and synchronously irradiates different regions of the sample of interest 20 through a plurality of optical paths, thus avoiding the quenching of the emission light in the same region due to the excessive power of the excitation lights when the plurality of light sources irradiate the same region and improving the sequencing quality. In the optomechanical system 10 of this embodiment, by arranging the movable platform device 200 and the illumination device 300 capable of emitting lights with two different wavelengths, the sample of interest 20 can be driven to move relative to the imaging device 400 during the sequencing process, so as to reduce the time for platform movement and image acquisition during the photographing process, thereby achieving a high-speed, high-throughput sequencing.

Specifically, referring to FIGs. 1 to 5 and FIG. 13, the imaging device 400 includes a beamsplitter assembly 410, a lens assembly 440, and a plurality of camera assemblies 420.

The lens assembly 440 is configured for projecting the excitation light to the sample of interest 20 to excite the sample of interest 20 to generate the emission light, and receiving the emission light generated by the sample of interest 20. In this embodiment, the lens assembly 440 is configured for projecting the excitation lights with two wavelengths to the sample of interest 20 to excite four bases A, T, C, and G bonded to the nucleic acid molecules of interest on the sample of interest 20 to generate four different emission light signals.

The beamsplitter assembly 410 includes a first dichroic mirror 411, a second dichroic mirror 412, a third dichroic mirror 413, a fourth dichroic mirror 414, and a fifth dichroic mirror 415. The first dichroic mirror 411 and the second dichroic mirror 412 are arranged on the optical axis of the lens assembly 440. The first dichroic mirror 411 is configured for reflecting the excitation light to lens assembly 440 to project the excitation light to the sample of interest 20 through the lens assembly 440, and configured for transmitting the emission light acquired by lens assembly 440 to the second dichroic mirror 412. The second dichroic mirror 412 is configured for reflecting the emission light from the first dichroic mirror 411 to the third dichroic mirror 413. The third dichroic mirror 413 is arranged in the optical path of the emission light reflected by the second dichroic mirror 412, and configured for receiving the emission light reflected by the second dichroic mirror 412, splitting the emission light into a first light beam with mixed wavelengths and a second light beam with mixed wavelengths, reflecting the first light beam with mixed wavelengths to the fourth dichroic mirror 414, and transmitting the second light beam with mixed wavelengths to the fifth dichroic mirror 415. The first light beam with mixed wavelengths includes a light beam with a first wavelength and a light beam with a second wavelength, and the second light beam with mixed wavelengths includes a light beam with a third wavelength and a light beam with a fourth wavelength. The light beams with the first wavelength, the second wavelength, the third wavelength, and the fourth wavelength respectively correspond to the four emission light signals generated by four bases A, T, C, and G bonded to the nucleic acid molecules of interest on the sample of interest 20.

The plurality of camera assemblies 420 are a first camera assembly 420-1, a second camera assembly 420-2, a third camera assembly 420-3, and a fourth camera assembly 420-4, each configured for acquiring an emission light with a corresponding wavelength.

In one embodiment, the first camera assembly 420-1 and the second camera assembly 420-2 are configured for acquiring two different emission light signals generated by the first region of the sample of interest 20 and forming an image; the third camera assembly 420-3 and the fourth camera assembly 420-4 are configured for acquiring two different emission light signals generated by the second region of the sample of interest 20 and forming an image. Through synchronous acquisition in different regions, mutual interference of different signals is avoided, and the imaging quality is improved.

Specifically, each of the camera assemblies includes a tube lens 421, a filter 424, and a TDI camera 422. The filter 424 is configured for allowing an emission light of one wavelength to enter the corresponding TDI camera 422. The tube lens 421 is positioned between the filter 424 and the TDI camera 422, and configured for converging the emission light filtered through the filter 421 to the TDI camera 422. The light-sensing surface of the TDI camera is adapted to the elongated spot. In the sequencing process, when the sample of interest 20 is driven to move by the movable platform device 200, the TDI camera 422 consecutively exposes and scans different regions in each line of the sample of interest 20 to acquire the emission light signals of the line, thus improving the signal acquisition efficiency and the sequencing throughput.

Further, the fourth dichroic mirror 414 is arranged on the optical path of the first light beam with mixed wavelengths, and configured for receiving the first light beam with mixed wavelengths, transmitting the light beam with the first wavelength of the first light beam with mixed wavelengths to the first camera assembly 420-1. That is, the light beam with the first wavelength sequentially passes through the filter 424 and the tube lens 421 of the first camera assembly 420-1, and is then received by the TDI camera 422 for imaging. Meanwhile, the fourth dichroic mirror 414 reflects the light beam with the second wavelength of the first light beam with mixed wavelengths to the second camera assembly 420-2 for imaging.

The fifth dichroic mirror 415 is arranged on the optical path of the second light beam with mixed wavelengths, and configured for receiving the second light beam with mixed wavelengths, transmitting the light beam with the third wavelength of the second light beam with mixed wavelengths to the third camera assembly 420-3 for imaging, and reflecting the light beam with the fourth wavelength of the second light beam with mixed wavelengths to the fourth camera assembly 420-4 for imaging. Therefore, the TDI cameras 422 in the four optical assemblies 420 are configured for simultaneously acquiring the emission light signals of the four bases A, T, C, and G, thus improving the sequencing efficiency.

By setting the third dichroic mirrors 413 to cooperate with the plurality of camera assemblies 420, the light beams split by the third dichroic mirrors 413 can be directed towards the plurality of camera assemblies 420, respectively, such that the plurality of camera assemblies 420 acquire corresponding emission light signals and form images. Specifically, the light beams are directed into the TDI cameras 422 after passing through the tube lens 421 and the filters 424, and are imaged by the TDI camera 422 to form images.

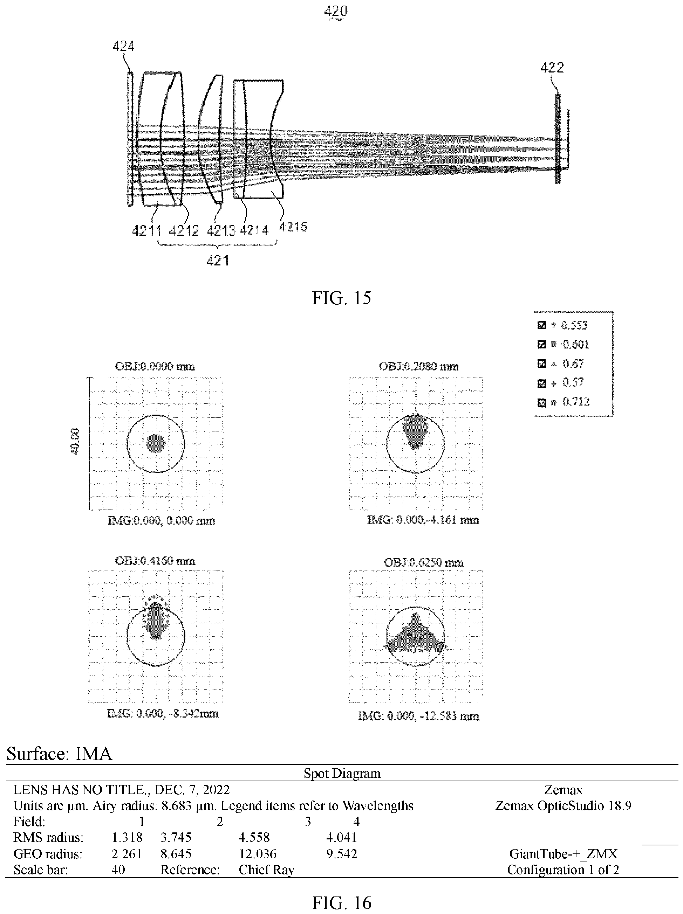

As shown in FIG. 15, in one embodiment, the tube lens 421 includes a first lens 4211, a second lens 4212, a third lens 4213, a fourth lens 4214 and a fifth lens 4215 that are arranged in sequence from an object side to an image side;

the first lens 4211 has a negative refractive power;
the second lens 4212 has a positive refractive power;
the third lens 4213 has a positive refractive power;

the fourth lens 4214 has a positive refractive power;
the fifth lens 4215 has a negative refractive power;
object-side surfaces and image-side surfaces of the first lens 4211, the second lens 4212, the third lens 4213, the fourth lens 4214, and the fifth lens 4215 are all spherical surfaces.

Specifically, the object-side surface of the first lens 4211 is convex at the optical axis of the first lens 4211, and the image-side surface of the first lens 4211 is concave at the optical axis of the first lens 4211. The object-side surface of the first lens 4211 has a curvature radius of 100 mm to 200 mm at the optical axis of the first lens 4211, and the image-side surface of the first lens 4211 has a curvature radius of 50 mm to 200 mm at the optical axis of the first lens 4211.

The object-side surface of the second lens 4212 is convex at the optical axis of the second lens 4212, and the image-side surface of the second lens 4212 is convex at the optical axis of the second lens 4212. The object-side surface of the second lens 4212 has a curvature radius of 50 mm to 200 mm at the optical axis of the second lens 4212, and the image-side surface of the second lens 4212 has a curvature radius of -100 mm to -500 mm at the optical axis of the second lens 4212.

The object-side surface of the third lens 4213 is convex at the optical axis of the third lens 4213, and the image-side surface of the third lens 4213 is planar at the optical axis of the third lens 4213. The object-side surface of the third lens 4213 has a curvature radius of 50 mm to 200 mm at the optical axis of the third lens 4213, and the image-side surface of the third lens 4213 has a curvature radius of 100 mm to 500 mm at the optical axis of the third lens 4213. The object-side surface of the fourth lens 4214 is planar at the optical axis of the fourth lens 4214, and the image-side surface of the fourth lens 4214 is convex at the optical axis of the fourth lens 4214. The object-side surface of the fourth lens 4214 has a curvature radius greater than 1000 mm at the optical axis of the fourth lens 4214, and the image-side surface of the fourth lens 4214 has a curvature radius of -100 mm to -500 mm at the optical axis of the fourth lens 4214.

The object-side surface of the fifth lens 4215 is concave at the optical axis of the fifth lens 4215, and the image-side surface of the fifth lens 4215 is concave at the optical axis of the fifth lens 4215. The object-side surface of the fifth lens 4215 has a curvature radius of -100 mm to -500 mm at the optical axis of the fifth lens 4215, and the image-side surface of the fifth lens 4215 has a curvature radius of 50 mm to -200 mm at the optical axis of the fifth lens 4215. The first lens 4211 has a thickness of 5 to 20 mm at the optical axis of the first lens 4211;

the second lens 4212 has a thickness of 5 to 20 mm at the optical axis of the second lens 4212;
the third lens 4213 has a thickness of 5 to 20 mm at the optical axis of the third lens 4213;
the fourth lens 4214 has a thickness of 5 to 20 mm at the optical axis of the fourth lens 4214;

the fifth lens 4215 has a thickness of 5 to 20 mm at the optical axis of the fifth lens 4215.

In one embodiment, the first lens 4211 and the second lens 4212 are cemented to form a first cemented lens set; the fourth lens 4214 and the fifth lens 4215 are cemented to form a second cemented lens set.

In one embodiment, the tube lens meets the relationship equation: $\frac{\Delta l'_{FC}}{\beta^2} \leq \frac{DOF}{5}$ , where the $\Delta l'_{FC}$ is the axial chromatic aberration, the $\beta$ is the magnification, the DOF is the depth of field, and $\Delta l'_{FC}$ is less than or equal to 100 mm. Through the combination of the first cemented lens set, the third lens, and the second cemented lens set, the aberration correction and apochromatic imaging can be achieved, and the field curvature is reduced. The tube lens 421 has a transmittance greater than 93%.

In this embodiment, the tube lens 421 consists of three lens sets, and the first cemented lens set and the second cemented lens set provide achromatic correction functionality, thus reducing the tolerance sensitivity. Specifically, the first cemented lens set is formed by cementing a biconvex lens and a concave-convex lens, and one convex surface of the biconvex lens is attached to the concave surface of the concave-convex lens; the second cemented lens set can be formed by cementing a biconcave lens and a plano-convex lens, and the convex surface of the plano-convex lens is attached to one concave surface of the biconcave lens so as to improving the field curvature. The symmetrical structure and the meniscus lens are also favorable for correcting the aberration. After tolerance analysis, it is confirmed that various comprehensive indexes of the tube lens 421 are within imaging requirements, MTF under a target usage line pair meets the requirements of algorithms on signal-to-noise ratio identification, the lenses in the tube lens 421 feature high machinability, and the field curvature, chromatic aberration, distortion, and the like all meet requirements of a sequencer on image quality.

In one embodiment, each of the camera assemblies includes the filter 424. The filter 424 is arranged on one side of the first lens 4211 distal to the second lens 4212. The distance between the first lens 4211 and the filter 424 may be 2 mm, and the filters 424 are mounted on parallel optical paths. As such, the influence on the image quality is reduced, and the optical filter can be used as an auxiliary reference positioning surface of the optical axis of the tube lens 421, thus facilitating the optical calibration.

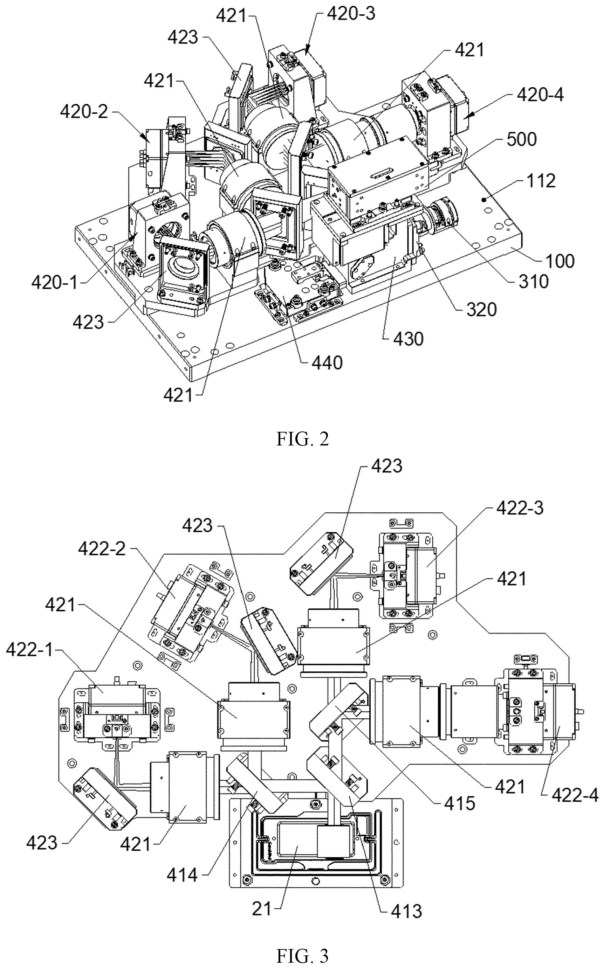

Referring to FIG. 3, in one embodiment, the illumination device 300 is configured for emitting two excitation lights with different wavelengths; the imaging device 400 includes two camera assemblies 420, and each of the camera assemblies 420 includes two TDI cameras 422. Each of the two TDI cameras 422 in each of the camera assemblies 420 is configured for receiving an emission light of one wavelength.

In this embodiment, the illumination device 300 can output through two optical paths. Each of the two optical paths is arranged in an independent portion of the corresponding illumination device 300, and does not affect each other. For example, the two optical paths may be lights with a wavelength of 532 nm and a wavelength of 640 nm, respectively, and the lights with different wavelengths emitted by the illumination device 300 may be set according to the actual sequencing requirements, which is not limited herein. By adapting the two TDI cameras 422 respectively to the emission light excited by the excitation lights of two wavelengths, the TDI cameras 422 can only receive the emission light with the wavelength corresponding to the preset value, so as to filter the lights with other wavelengths, thereby improving the sequencing accuracy and the sequencing efficiency of the optomechanical system 10.

Referring to FIG. 3, in a specific embodiment, the four TDI cameras 422 are a G camera 422-1, an A camera 422-2, a C camera 422-3, and a T camera 422-4, and the illumination device 300 synchronously emits a red laser light and a green laser light to two regions. The A camera 422-2 and the C camera 422-3 can respectively acquire two emission light signals with different wavelengths excited by the red laser light, and the G camera 422-1 and the T camera 422-4 can respectively acquire two emission light signals excited by the green laser light.

Further, in one embodiment, the camera assembly 420 includes mirrors 423, and one mirror 423 is arranged between the G camera 422-1 and the tube lens 421, between the A camera 422-2 and the tube lens 421, and between the C camera 422-3 and the tube lens 421, such that the lights can be transmitted to the corresponding camera 422 through the tube lens 421, thus making the overall structure of the imaging device 400 more compact.

Specifically, the mirror 423 of the C camera 422-3, the mirror 423 of the G camera 422-1, and the mirror 423 of the A camera 422-2 are all adjustable in pitch angle. By adjusting the mirrors 423, the lights can be directed perpendicular to the light-sensing surfaces of the G camera 422-1, the A camera 422-2, and the C camera 422-3. Meanwhile, the C, G, and A cameras are adjustable in three degrees of freedom, and by adjusting the three degrees of freedom of the camera 422, the center of the light-sensing surface of the camera 422 can be made coincide with the center of the optical path. The T camera 422-4 may be adjustable in four degrees of freedom, and by adjusting the four degrees of freedom of the T-camera 422-4, the center of the light-sensitive surface of the T camera 422-4 can be made coincide with the center of the optical path, while the light is directed perpendicular to the light-sensitive surface of the T camera 422-4.

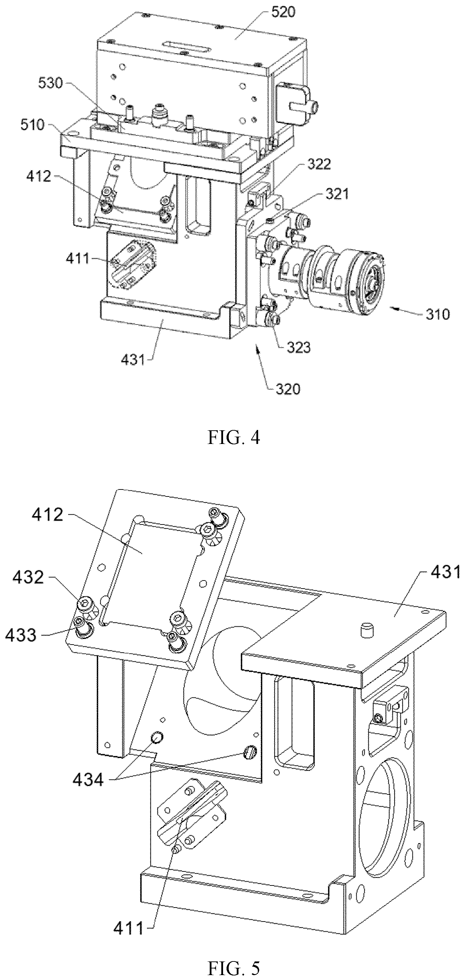

Further, referring to FIG. 2 and FIG. 4, the imaging device 400 further includes an optical path turning assembly 430. The optical path turning assembly 430 includes a first base 431. The first base 431 is arranged on the mounting surface 112. The first dichroic mirror 411 is adjustably connected to the first base 431. The illumination device 300 is connected to the first base 431, and an excitation light output port of the illumination device 300 is oriented towards the first dichroic mirror 411. The first dichroic mirror 411 is configured for projecting the lights emitted by the illumination device 300 on the sample of interest 20 through the lens assembly 440 to excite the sample of interest 20 to generate the emission lights, and for transmitting the emission light generated by the sample of interest 20 to the second dichroic mirror 412.

In this embodiment, by setting the first dichroic mirror 411 to cooperate with the illumination device 300 and the imaging device 400, the excitation lights emitted by illumination device 300 may be transmitted to the sample of interest 20 after passing through the first dichroic mirror 411, and the emission light generated by the sample of interest 20 may be transmitted to the second dichroic mirror 412 after passing through the first dichroic mirror 411. In a preferred embodiment, the first dichroic mirror 411 may also be adjustably arranged relative to first base 431, and by adjusting the first dichroic mirror 411, the propagation direction of the excitation light and/or the emission light may be adjusted, such that excitation light is incident perpendicularly on the lens assembly 440, and/or the emission light is aligned with or perpendicular to the center of the light-sensing surface of the TDI camera 422. Specifically, referring to FIG. 5, the imaging device 400 further includes a first adjusting assembly. The first adjusting assembly includes a first adjusting member 432 and a first fixing member 433. The first adjusting member 432 is in a threaded connection with the second dichroic mirror 412 and abuts against the first base 431. The first fixing member 433 is arranged through the second dichroic mirror 412 and is connected to the first base 431.

When the imaging device 400 of this embodiment is used, the second dichroic mirror 412 can be driven to move toward or away from the first base 431 by rotating the first adjusting member 432 (certainly, any one or more sides of the second dichroic mirror 412 can be driven to move toward or away from the first base 431) to adjust the relative position of the second dichroic mirror 412 on the first base 431. After the adjustment is accomplished, the second dichroic mirror 412 can be fastened on the first base 431 by rotating the first fixing member 433. On the contrary, when the adjustment of the second dichroic mirror 412 is required, the second dichroic mirror 412 can be released simply by reversely rotating the first fixing member 433, featuring a simple structure and ease of operation. Further, the optical path turning assembly 430 further includes a first positioning insert block 434. The first positioning insert block 434 is detachably connected to the first base 431 and is arranged on the side towards the first dichroic mirror 411. The first adjusting member 432 abuts against the first positioning insert block 434.

By arranging the first positioning insert block 434 on the first base 431 to cooperate with the first adjusting member 432, when the first adjusting member 432 and/or the first positioning insert block 434 are abraded, the maintenance cost is reduced by simply replacing the first adjusting member 432 and/or the first positioning insert block 434. Also, the first positioning insert block 434 and the first base 431 may be made of different materials. For example, the first positioning insert block 434 is made of an abrasion-resistant material to reduce the frictional resistance between the two in use of the first adjusting member 432, and the first base 431 is made of a high-strength material. As such, the manufacturing cost-efficiency of the optical path turning assembly 430 may also be improved.

In one embodiment, the first positioning insert block 434 may include a point positioning insert (equipped with an arc slot therein), a line positioning insert (equipped with a bar slot or V-shaped slot therein), and a plane positioning insert to cooperate with the first adjusting member 432. The point positioning insert can limit the rectilinear movement in the degrees of freedom in two directions between the two; the line positioning insert, by cooperating with the point positioning insert, can limit the parallel rotation degree of freedom of the second dichroic mirror 412 relative to the first base 431; the plane positioning insert can support the first adjusting member 432. The cooperation of the three can effectively improve adjustment precision and assembly stability between the second dichroic mirror 412 and the first base 431.

Referring to FIGs. 4 and 5, in one embodiment, the second dichroic mirror 412 is connected to the first base 431, arranged downstream of the first dichroic mirror 411 along the optical axis of the lens assembly 440, and configured for receiving the emission light transmitted by the first dichroic mirror 411 and reflecting the emission light to the third dichroic mirror 413.

In some embodiments, the optomechanical system 10 further includes an automatic focusing device 500. The automatic focusing device 500 is connected to the first base 431. The automatic focusing device is configured for emitting a focusing light beam, such that the focusing light beam sequentially passes through the second dichroic mirror 412, the first dichroic mirror 411, and the lens assembly 440, and then irradiates the sample of interest 20. The automatic focusing device 500 is further configured for receiving the focusing light beam reflected by the sample of interest 20 along the original path. The first dichroic mirror 411 and the second dichroic mirror 412 are configured for transmitting the focusing light beam emitted by the automatic focusing device and the focusing light beam reflected by the sample of interest 20.

With this arrangement, after the emission light generated by the sample of interest 20 and the focusing light beam reflected by the sample of interest 20 pass through the first dichroic mirror 411, they may be transmitted to the imaging device 400 and the automatic focusing device 500, respectively, by the second dichroic mirror 412. In this embodiment, the first dichroic mirror 411 and the second dichroic mirror 412 are sequentially arranged on the optical axis of the lens assembly 440 from bottom to top. The first dichroic mirror 411 is positioned between the second dichroic mirror 412 and the sample of interest 20. After the emission light generated by the sample of interest 20 passes through the second dichroic mirror 412, the second dichroic mirror 412 can transmit the light to the camera assembly 420 after changing the direction by 90° and transmit the focusing light beam, after passing through the second dichroic mirror 412, to the automatic focusing device 500. This arrangement features a compact overall structure and good effects of use.

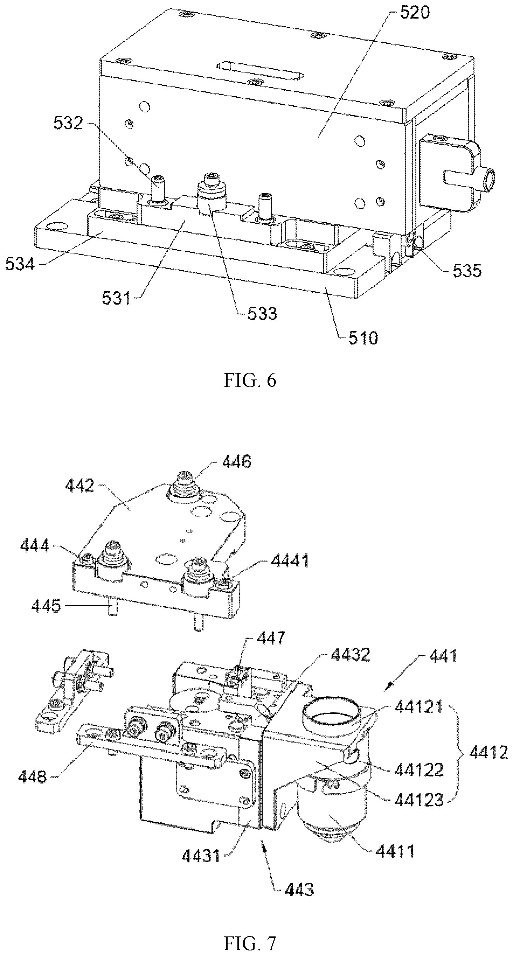

The automatic focusing device 500 is configured for emitting the focusing light beam to the sample of interest 20 and receiving the focusing light beam reflected by the sample of interest 20, so as to detect whether the sample of interest 20 is positioned on the focal plane of the lens assembly 440. Specifically, referring to FIGs. 4 and 6, the automatic focusing device 500 includes a second base 510, a focusing assembly 520, and a second adjusting assembly 530. The second base 510 is connected to the first base 431. The focusing assembly 520 is connected to the second base 510. The second adjusting assembly 530 is configured for adjusting relative positions between the focusing assembly 520 and the second base 510.

In the present embodiment, by arranging the second adjusting assembly 530 to connect the second base 510 and the focusing assembly 520, the second base 510 is configured in a fixed connection with the bracket device 100. The second adjusting assembly 530 can adjust the focusing assembly 520 relative to the second base 510, including, but not limited to, movement adjustment, pitch adjustment, and rotation adjustment. Certainly, in a preferred embodiment, the second adjusting assembly 530 can perform movement adjustment and pitch adjustment on the focusing assembly 520.

Further, referring to FIG. 6, the second adjusting assembly 530 includes a first movable seat 531 and a second adjusting member 532. The first movable seat 531 is movably arranged relative to the second base 510. The focusing assembly 520 is arranged on the first movable seat 531. The second adjusting member 532 abuts against the second base 510 and is connected to the first movable seat 531, so as to perform pitch adjustment on the focusing assembly 520 relative to the second base 510, i.e., to adjust the pitch angle of the focusing assembly 520 relative to the second base 510 when the second adjusting member 532 is operated.

When the optomechanical system 10 of this embodiment is used, if the focusing assembly 520 needs to be adjusted in pitch angle, the second adjusting member 532 is operated to abut against the second base 510 to drive the first movable seat 531 to perform pitch adjustment. In this embodiment, the number of the second adjusting member 532 is more than one, and the second adjusting members 532 are arranged around the first movable seat 531 and in threaded connections with the first movable seat 531. The first movable seat 531 can be driven to move in pitch angle relative to the second base 510 by rotating the second adjusting members 532.

Referring to FIG. 6, the second adjusting assembly 530 further includes a second fixing member 533. The second fixing member 533 is arranged through the first movable seat 531 and in a threaded connection with the second base 510. As such, when the focusing assembly 520 is adjusted to a preset position, the first movable seat 531 and the second base 510 can be fixedly connected by fastening the second fixing member 533.

In one embodiment, the second adjusting assembly 530 includes a second movable seat 534 and a third adjusting member 535. The second movable seat 534 is movably arranged relative to the second base 510. The focusing assembly 520 and the first movable seat 531 are arranged on the second movable seat 534. The third adjusting member 535 is separately connected to the second movable seat 534 and the second base 510.

In this embodiment, the third adjusting member 535 is in a threaded connection with the second movable seat 534, and is rotatably connected to the second base 510. The second movable seat 534 can be driven to move relative to the second base 510 by rotating the third adjusting member 535, so as to perform movement adjustment on the focusing assembly 520 relative to the second base 510.

Specifically, referring to FIGs. 2 and 7, the imaging device 400 further includes the lens assembly 440. The lens assembly 440 includes an objective lens structure 441 and a third base 442. The third base 442 is connected to the bracket device 100. The objective lens structure 441 is connected to the third base 442 and arranged between the sample of interest 20 and the first dichroic mirror 411.

Referring to FIGs. 7 and 12, in one embodiment, the objective lens structure 441 includes an objective lens 4411 and a mounting bracket 4412. The objective lens 4411 is configured for projecting the excitation light emitted by the light source to the sample of interest 20 and acquiring the emission light signal generated by the sample of interest 20. The emission light signal is received by the TDI camera 422 after passing through the tube lens 421 and the filter 424 to form an image. The objective lens 4411 is provided with a first light-transmitting region 4411a and a second light-transmitting region 4411b. The first light-transmitting region 4411a is configured for projecting the excitation light emitted by the first light source 301 into the first region of the sample of interest 20 and receiving the emission light generated in the first region. The second light-transmitting region 4411b is configured for projecting the excitation light emitted by the second light source 302 into the second region of the sample of interest 20 and receiving the emission light generated in the second region. That is, the objective lens 4411 synchronously projects the excitation lights with different wavelengths to different regions of the sample of interest 20 and simultaneously receives the emission lights generated by the sample of interest 20 in the first region and the second region, thus avoiding the quenching of the emission light in the same region due to the excessive power of the excitation lights when the plurality of light sources irradiate the same region.

Since the distance between sites of the nucleic acid molecules of interest on the sample of interest 20 is typically several hundred nanometers, the objective lens is designed to have a high numerical aperture (NA) according to the Rayleigh criterion. In this embodiment, the objective lens has a numerical aperture greater than 0.75, which can significantly improve the spatial resolution of the object and improve the image quality. In addition, the objective lens is designed to have a large field of view in consideration of the fact that the imaging region of the optomechanical system is an elongated region with a high aspect ratio. In this embodiment, the objective lens has a field of view greater than 1.2 mm. That is, the objective lens has a high numerical aperture and a large field of view.

The mounting bracket 4412 and the third base 442 are movable relative to each other, and the objective lens 4411 is detachably connected to the mounting bracket 4412.

In this embodiment, the objective lens 4411 and the mounting bracket 4412 are detachably connected to each other, which can improve convenience in combining the two and facilitate the assembly and later maintenance. Specifically, the mounting bracket 4412 includes a shade barrel 44121, a guide barrel 44122, and a connecting plate 44123 that are connected in sequence. The connecting plate 44123 is detachably connected to the third base 442. The shade barrel 44121 is positioned on the side of the guide barrel 44122 distal to the sample of interest 20 and configured for shading the imaging device 400 against stray light. The guide barrel 44122 can guide the transmitted light. As shown in FIG. 7, in the embodiment, an L-shaped slot is formed on the outer circumferential wall of the bottom of the guide barrel 44122, and a connecting protrusion corresponding to the L-shaped slot can be formed on the outer wall of the objective lens 4411. When the guide barrel 44122 and the objective lens 4411 are assembled, the objective lens 4411 may be connected upwards to the L-shaped slot of the guide barrel 44122, and then the objective lens 4411 is rotated to make the connecting protrusion hang in the L-shaped slot of the guide cylinder 44122, which features the convenience of assembly and a compact overall structure.

Further, the lens assembly 440 further includes a lifting structure 443. The lifting structure 443 is separately connected to the third base 442 and the objective lens structure 441 and configured for driving the objective lens structure 441 to move up and down relative to the third base 442.

In this embodiment, the automatic focusing device 500 is coupled to the lens assembly 440 through the optical path. By setting the lifting structure 443 to cooperate with the objective lens structure 441, during the operation of the optomechanical system 10, the focusing light beam information acquired by the automatic focusing device 500 can be fed back to the lifting structure 443, and the distance between the objective lens 4411 and the sample of interest 20 can be adjusted, so as to position the sample of interest 20 on the focal plane of the objective lens and ensure the image definition. Certainly, in some embodiments, the lifting structure 443 may also be adjusted or controlled manually by, e.g., a threaded connection, a gear and rack combination structure, a guide rod, or a slide rail structure, etc. The positions between the objective lens 4411 and the sample of interest 20 can be adjusted through manual adjustment.

Furthermore, the lifting structure 443 includes a driving member 4431 and a stopper 4432. The stopper 4432 is connected to the objective structure 441, and is at least partially positioned on the top of the third base 442. The driving member 4431 is separately connected to the objective lens structure 441 and the third base 442 and configured for driving the objective lens structure 441 to move.

With this arrangement, when the driving member 4431 drives the objective lens structure 441 to descend to the limit position, the stopper 4432 can abut against the third base 442 to limit the movement of the objective lens structure 441, so as to prevent the objective lens structure 441 from being detached from the lifting structure 443, and improve the reliability of the optomechanical system 10.

In one embodiment, the lens assembly 440 includes a first spring 447. The first spring 447 is arranged on the lifting structure and configured for applying an elastic force to the objective lens structure 441 when the lifting structure 443 drives the objective lens structure 441 to move. By providing the first spring 447, the weight of the load can be balanced by adjusting the first spring 447.

In this embodiment, the elastic force generated by the first spring 447 is balanced with the load on the Z-axis. When the load changes, the first spring 447 can be expanded and contracted to ensure the balance with the load, so as to provide better dynamic response performance to movement on the Z-axis, higher precision, and stronger interference resistance.

Further, referring to FIG. 7, the lens assembly 440 further includes a third adjusting assembly 444. The third adjusting assembly 444 is connected to the third base 442, abuts against the bracket device 100, and is configured for driving the third base 442 to move relative to the bracket device 100.

In the optomechanical system 10 of this embodiment, the third adjusting assembly 444 may be in a threaded connection with the third base 442. Furthermore, referring to FIGs. 7 to 9, the third adjusting assembly 444 includes a second positioning insert block 4442 and a fourth adjusting member 4441. The second positioning insert block 4442 is detachably connected to the bracket device 100. The fourth adjusting member 4441 is in a threaded connection with the third base 442, and abuts against the second positioning insert block 4442. The fourth adjusting member 4441 can be driven by rotating to abut against the bracket device 100, so as to drive the third base 442 to adjust relative to the bracket device 100, such that the excitation light can be incident perpendicularly on the sample of interest 20 after passing through the objective lens structure 441.

By arranging the second positioning insert block 4442 on the bracket device 100 to cooperate with the fourth adjusting member 4441, when the fourth adjusting member 4441 and/or the second positioning insert block 4442 are abraded, the maintenance cost is reduced by simply replacing the fourth adjusting member 4441 and/or the second positioning insert block 4442. Also, the second positioning insert block 4442 and the bracket device 100 may be made of different materials. For example, the second positioning insert block 4442 is made of an abrasion-resistant material to reduce the frictional resistance between the two in use of the fourth adjusting member 4441, and the bracket device 100 is made of a high-strength material. As such, the manufacturing cost-efficiency of the bracket device 100 may also be improved.

In one embodiment, the second positioning insert block 4442 may include a point positioning insert (equipped with an arc slot therein), a line positioning insert (equipped with a bar slot or V-shaped slot therein), and a plane positioning insert to cooperate with the fourth adjusting member 4441. The point positioning insert can limit the rectilinear movement in the degrees of freedom in two directions between the two; the line positioning insert, by cooperating with the point positioning insert, can limit the parallel rotation degree of freedom of the third base 442 relative to the bracket device 100; the plane positioning insert can support the fourth adjusting member 4441. The cooperation of the three can effectively improve adjustment precision and assembly stability between the third base 442 and the bracket device 100.

Referring to FIG. 7, in one embodiment, the lens assembly 440 further includes a third fixing member 445 and a second spring 446. The third fixing member 445 is separately connected to the third base 442 and the bracket device 100. The second spring 446 sleeves the third fixing member 445 and is arranged through the third base 442 and connected to the bracket device 100 to drive the third base 442 to have a tendency of moving towards the bracket device 100.

In this embodiment, the lens assembly 440 further includes a connection seat 448. The connection seat 448 is fixed on the bracket device 100, and the third base 442 and the connection seat 448 are connected via the third fixing member 445. During the process of adjusting the height of the third base 442, by setting the second spring 446 to cooperate with the third base 442, the third base 442 is provided with a reaction force when moving towards the bracket device 100, so as to balance the abutting force of the fourth adjusting member 4441, thereby improving the adjustment precision of the lens assembly 440.

Specifically, referring to FIG. 4, the illumination device 300 includes an illumination assembly 310 and a fourth adjusting assembly 320. The fourth adjusting assembly 320 includes at least one of a linear adjusting mechanism 321, a rotational adjusting mechanism 322, and a pitch adjusting mechanism 323, and is separately connected to the illumination assembly 310 and the bracket device 100 and configured for driving the illumination assembly 310 to move relative to the bracket device 100.

In this embodiment, by arranging the fourth adjusting assembly 320 having at least one of the linear adjusting mechanism 321, the rotational adjusting mechanism 322, and the pitch adjusting mechanism 323 in connection with the illumination assembly 310, the linear adjusting mechanism 321 can perform linear movement adjustment on the illumination assembly 310 relative to the bracket device 100, the rotational adjusting mechanism 322 can perform rotational adjustment on the illumination assembly 310 relative to the bracket device 100, and the pitch adjusting mechanism 323 can perform pitch adjustment on the illumination assembly 310 relative to the bracket device 100. As such, the fourth adjusting assembly 320 can adjust the illumination position of the illumination assembly 310, featuring good effects of use.

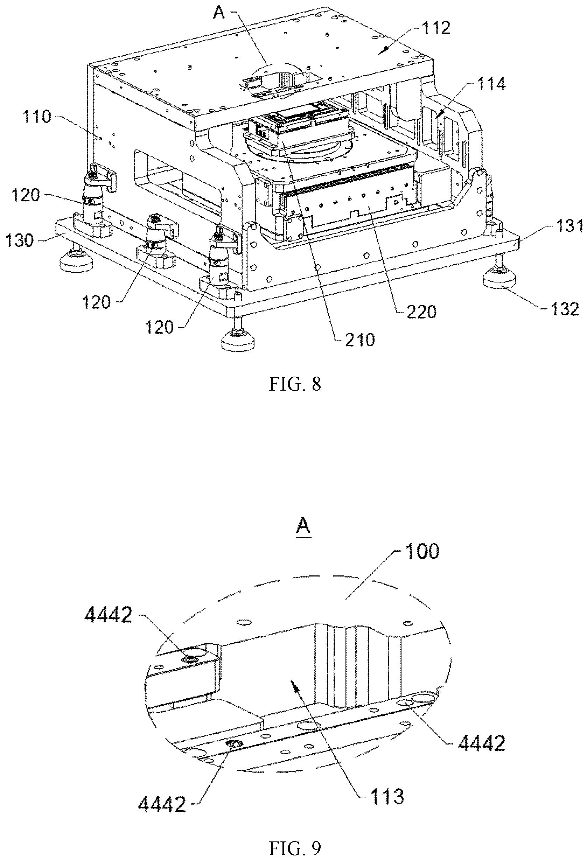

Referring to FIGs. 8 to 10, the movable platform device 200 includes a carrier structure 210 and a movable carrier platform 220. The carrier structure 210 is provided with a receiving slot 2111 configured for receiving the sample of interest 20. The carrier structure 210 is arranged on the movable carrier platform 220. The movable carrier platform 220 is connected to the bracket device 100, arranged in the cavity 111, and configured for driving the carrier structure 210 to conduct at least one of linear motion and rotatory motion relative to the bracket device 100.

With this arrangement, after the optomechanical system 10 finishes the scanning, the movable carrier platform 220 may drive the sample of interest 20 to move out of the cavity 111, so as to facilitate the operator to replace the sample of interest 20. When the optomechanical system 10 performs a linear scanning operation, the movable carrier platform 220 may further drive the carrier structure 210 to rotate and/or translate, so as to ensure that, for example, a reference line on the sample of interest 20 is parallel to the scanning direction of the imaging device 400, thereby improving the scanning effect of the optomechanical system 10.

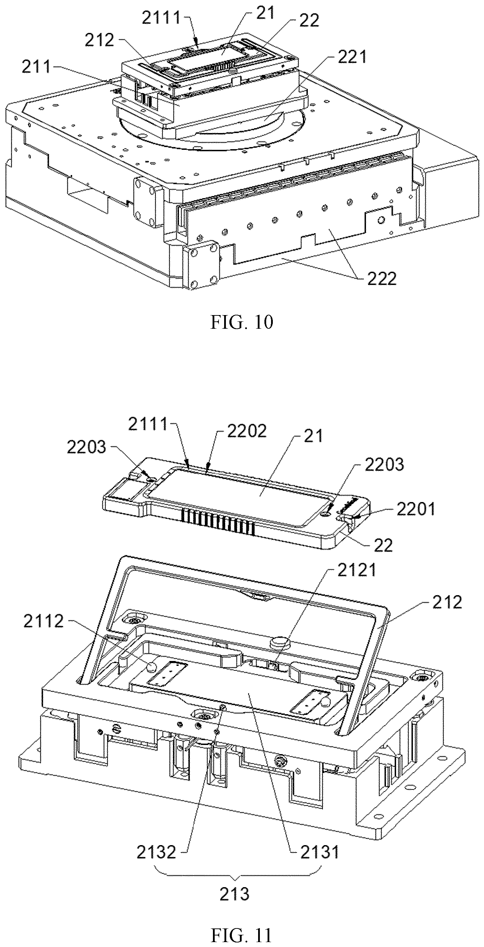

Referring to FIGs.10 and 11, in one embodiment, the carrier structure 210 includes a carrier stage 211 and a locking member 212. The locking member 212 is movably connected to the carrier stage 211. The receiving slot 2111 is arranged on the carrier stage 211. The sample of interest 20 is provided with a positioning slot 2201. The locking member 212 is configured for snap-fitting with the positioning slot 2201 to fix the sample of interest 20 in the receiving slot 2111.

When the carrier structure 210 of this embodiment is used to carry the sample of interest 20, the locking member 212 is firstly turned over to open the receiving slot 2111 on the carrier stage 211. Then the sample of interest 20 is placed in the receiving slot 2111, and the locking member 212 is turned over to correspond to the positioning slot 2201 of the sample of interest 20 until the locking member 212 is in snap-fit with the positioning slot 2201, so as to secure the mounting of the sample of interest 20 to the carrier stage 211 and improve the stability of fixing the sample of interest 20 to the carrier stage 211. When the sample of interest 20 needs to be removed, the locking member 212 can be reversely turned over to release the sample of interest 20, featuring a simple structure and ease to operate. Specifically, the carrier stage 211 is further provided with a snap fastener 2121. When the locking member 212 is pressed into the positioning slot 2201, the snap fastener 2121 is in snap-fit with the locking member 212 to fix the locking member 212.

Specifically, the sample of interest 20 includes a body 21 and a carrier frame 22. The carrier frame 22 is provided with a mounting slot 2202 for accommodating the body 21, and the positioning slot 2201 is arranged on the outer wall of the carrier frame 22.

Through arranging the carrier frame 22 to carry the body 21, when the sample of interest 20 is fixed on the carrier stage 211 via the locking member 212, the direct contact between the locking member 212 and the body 21 and damage to the body 21 can be avoided, thus featuring good effects of use.

Further, the carrier structure 210 includes the carrier stage 211 and a temperature controller 213. The receiving slot 2111 is arranged on the carrier stage 211. The temperature controller 213 includes a heating stage 2131 and a temperature sensor 2132. The heating stage 2131 is arranged at the bottom of the receiving slot 2111. The temperature sensor 2132 is connected to the carrier stage 211 and configured for acquiring the temperature of the sample of interest 20.

In this embodiment, by setting the temperature controller 213 to cooperate with the sample of interest 20, the heating stage 2131 can heat the reaction solution in the fluid channel of the sample of interest 20, and the temperature sensor 2132 can acquire the temperature of the sample of interest 20 in real time to perform feedback control on the heating stage 2131.

Specifically, the top of the carrier stage 211 is provided with a positioning part 2112, and the bottom of the carrier frame 22 is provided with a positioning hole 2203 mating with the positioning part 2112.

During the process of placing the sample of interest 20 in the receiving slot 2111, the positioning part 2112 may mate with the positioning hole 2203 of the carrier frame 22, so as to position the loading of the sample of interest 20. In a preferred embodiment, the positioning part 2112 is of a spheric structure, such that, when the positioning part 2112 mates with the sample positioning hole 2203, the sample of interest 20 can be guided by the arc surface, so as to improve the convenience of loading the sample of interest 20.

Specifically, referring to FIG. 10, the movable carrier platform 220 includes a rotary stage 221 and a movable carrier stage 222. The rotary stage 221 is separately connected to the movable carrier stage 222 and the carrier structure 210, and configured for driving the carrier structure 210 to rotate relative to the bracket device 100. The movable carrier stage 222 is configured for driving the carrier structure 210 to move along at least one direction relative to the imaging device 400, so as to allow the imaging device 400 to image different regions of the sample of interest 20.

Further, referring to FIGs. 8 and 9, the bracket device 100 includes a frame 110, a vibration damping structure 120, and a bottom plate structure 130. The frame 110 is arranged apart from the bottom plate structure 130 and is positioned over the bottom plate structure 130. The vibration damping structure 120 is separately connected to the frame 110 and the bottom plate structure 130. The frame 110 is provided with an accommodation hole 113 in separate communication with the cavity 111 and the mounting surface 112. The lens assembly 440 is accommodated in the accommodation hole 113.

In the embodiment, the frame 110 is levitated over the top of the bottom plate structure 130 and the two are connected via the vibration damping structure 120, such that the two are connected in a non-rigid manner. This arrangement can reduce the vibration conducted by the bottom plate structure 130 to the frame 110, thereby improving the scanning precision of the imaging device 400 and the transportation smoothness of the movable platform device 200. In addition, the vibration damping structure 120 may be arranged outside the frame 110, so as to lower the center of gravity of the frame 110, reduce the transmission of high-frequency vibration outside the frame 110, and improve the precision and stability of the optomechanical system 10.

Furthermore, the frame 110 is provided with a plurality of slots 114. Such an arrangement can reduce the overall weight of the optomechanical system 10, improve the natural frequency of the optomechanical system 10, and avoid low-frequency resonance.

Specifically, the bottom plate structure 130 includes a bottom plate body 131 and supports 132. The bottom plate body 131 contacts with the bottom surface or the benchtop through the supports 132. The support 132 includes a support leg and a vibration damping member sleeving the support leg. The vibration damping member may be, for example, a silica gel pad. The vibration damping member, by sleeving the support leg, can further reduce the vibration conduction capability of the bracket device 100 and thus further improve the precision and the stability of the optomechanical system 10.

Referring to FIG. 16, FIG. 16 is a spot diagram of the optomechanical system 10 in different fields of view in this embodiment, where the spot diagram illustrates the spot patterns of the fields of view on the object side observed on the image side. The spot diagram has a diffraction-limited circle, the radius of which is the Airy radius. As shown in FIG. 16, the spots of the fields of view are all within or close to the diffraction-limited circle. The spot sizes reach the diffraction limit in the fields of view, and a better image quality is achieved.

The present disclosure further provides a sequencing system, including the optomechanical system 10 in any one of the above embodiments.

In the sequencing system of this embodiment, by arranging the optomechanical system 10 in any one of the above embodiments, the optomechanical system 10 can, by setting the movable platform device 200 to cooperate with the sample of interest 20, drive the sample of interest 20 to move relative to the imaging device 400 in the sequencing process, so as to meet different sequencing requirements such as line feed scanning, etc. After the sequencing is completed, the sample of interest 20 can be removed, thus facilitating the removal and loading of the sample of interest 20 and featuring the convenience of use. Also, as the illumination device 300 emits two lights with different wavelengths to the sample of interest 20, synchronous sequencing in different regions through multiple optical paths can be achieved during the sequencing process, so as to avoid the quenching of the emission light due to the excessive power of the excitation lights in the focused region and ensuring the sequencing successful rate. The sample of interest 20 can be driven to move relative to the imaging device 400 during the sequencing process, so as to reduce the time for platform movement and image acquisition during the photographing process, thereby achieving a high-speed, high-throughput sequencing. In the description of the embodiments of the present disclosure, it should be noted that orientational or positional relationships indicated by terms such as "central", "longitudinal", "transverse", "upper", "lower", "front", "rear", "left", "right", "vertical", "horizontal", "top", "bottom", "inner", "outer", and the like are those shown on the basis of the accompanying drawings, and are merely intended to facilitate and simplify the description of the embodiments of the present disclosure rather than indicate or imply that the indicated device or element must have a specific orientation and be configured and operated according to the specific orientation. Such relationships should not be construed as limiting the embodiments of the present disclosure. In addition, the terms "first", "second", and "third" are used for descriptive purposes only and should not be construed as indicating or implying relative importance.

In the description of the embodiments of the present disclosure, it should be noted that unless otherwise clearly specified and defined, the terms "link" and "connect" should be interpreted in their broad sense. For example, the connection may be a fixed connection, detachable connection, or integral connection; a mechanical connection or electric connection; or a direct connection or indirect connection through an intermediate. For those of ordinary skill in the art, the specific meanings of the terms described above in the embodiments of the present disclosure can be interpreted according to specific conditions.

In the embodiments of the present disclosure, unless otherwise clearly specified and defined, a first feature being "above" or "under" a second feature may refer to that the first feature and the second feature are in direct contact, or the first feature and the second feature are in indirect contact through an intermediate. Also, a first feature being "on", "over", and "above" a second feature may refer to that the first feature is right above or obliquely above the second feature, or simply mean that the first feature is at a higher vertical position than the second feature. A first feature being "under", "beneath", and "below" a second feature may refer to that the first feature is right below or obliquely below the second feature, or simply mean that the first feature is at a lower vertical position than the second feature. Reference throughout this specification to "an embodiment", "one embodiment", "another embodiment", "some embodiments", "an example", "a specific example", "another example", or "some examples ", means that a particular feature, structure, material, or characteristic described in connection with the embodiment or example is included in at least one embodiment or example of the present disclosure. In the specification, the schematic expression of the terms described above does not necessarily refer to the same embodiment or example. Furthermore, the particular features, structures, materials, or characteristics may be combined in any suitable manner in any one or more embodiments or examples. In addition, in the absence of contradiction, those skilled in the art can combine the different embodiments or examples described in this specification or combine the features of different embodiments or examples.

Finally, it should be noted that: the above embodiments are only intended to describe, rather than limit, the technical solutions of the present disclosure. Although the present disclosure has been described in detail with reference to the foregoing embodiments, it should be understood by those of ordinary skill in the art that: modifications can still be made to the technical solutions described in the foregoing embodiments, or some of the technical features can be equivalently substituted; and these modifications or substitutions do not depart the nature of the corresponding technical solutions from the spirit and scope of the technical solutions of the embodiments of the present disclosure. The present invention may be characterized by the following items.

1. An optomechanical system, comprising:

an illumination device, configured for emitting an excitation light to irradiate a sample of interest so as to excite the sample of interest to generate an emission light;
an imaging device, configured for acquiring the emission light and forming an image; and
a movable platform device, configured for driving the sample of interest to translate and/or rotate relative to the imaging device, so as to enable the imaging device to consecutively acquire the emission light.

2. The optomechanical system according to item 1, wherein
the illumination device comprises one or more light sources, and the light sources are configured for emitting a plurality of excitation lights with different wavelengths to respectively irradiate different regions of the sample of interest.

3. The optomechanical system according to item 2, wherein
the plurality of excitation lights with different wavelengths respectively irradiate different regions of the sample of interest along different illumination optical paths.

4. The optomechanical system according to any one of items 1-3, wherein

the illumination device comprises a shaping lens set;
the shaping lens set is positioned on an illumination optical path of the excitation light;
the shaping lens set is configured for shaping the excitation light in a first direction and a second direction perpendicular to the first direction to adapt a shape and/or size of a spot of the excitation light to an imaging region of the imaging device.

5. The optomechanical system according to item 4, wherein

the illumination device comprises a collimation lens;
the collimation lens is positioned on an upstream or downstream optical path of the shaping lens set.

6. The optomechanical system according to any one of items 2-5, wherein
the light sources comprise a first light source and a second light source; the first light source and the second light source are respectively configured for emitting an excitation light with a first wavelength and an excitation light with a second wavelength; the excitation light with the first wavelength irradiates a first region of the sample of interest along a first illumination optical path to form a first spot, and the excitation light with the second wavelength irradiates the second region of the sample of interest along a second illumination optical path to form a second spot; the first spot does not overlap with the second spot.

7. The optomechanical system according to item 6, wherein

the shaping lens set comprises a first shaping lens set and a second shaping lens set;
the first shaping lens set is arranged on the first illumination optical path, and the excitation light with the first wavelength irradiates the first region of the sample of interest after being shaped by the first shaping lens set to form an elongated first spot;
the second shaping lens set is arranged on the second illumination optical path, and the excitation light emitted by
the second light source irradiates the second region of the sample of interest after being shaped by the second shaping lens set to form an elongated second spot.

8. The optomechanical system according to item 7, wherein
the first shaping lens set and the second shaping lens set each comprise a first cylindrical lens and a second cylindrical lens; the first cylindrical lens and the second cylindrical lens are respectively configured for zooming the excitation light in the first direction and the second direction to adapt the shape and/or size of the spot of the excitation light to the imaging region of the imaging device.

9. The optomechanical system according to item 7 or 8, wherein

the collimation lens comprises a first collimation lens and a second collimation lens;
the first collimation lens is positioned on an upstream or downstream optical path of the first shaping lens set;
the second collimation lens is positioned on an upstream or downstream optical path of the second shaping lens set.

10. The optomechanical system according to any one of items 4-9, wherein end faces of the illumination device for outputting the excitation light of the first wavelength and the excitation light of the second wavelength are rectangular.

11. The optomechanical system according to any one of items 1-10, wherein

the imaging device comprises a lens assembly, a beamsplitter assembly, and a plurality of camera assemblies;
the lens assembly is configured for projecting the excitation light to the sample of interest, acquiring the emission light generated by the sample of interest, and projecting the emission light to the beamsplitter assembly;
the beamsplitter assembly is configured for receiving the emission light acquired by the lens assembly, splitting the emission light, and projecting the emission light into the plurality of camera assemblies;
the plurality of camera assemblies are configured for acquiring the emission light and forming the image.

12. The optomechanical system according to item 11, wherein

the plurality of camera assemblies comprises four camera assemblies, i.e., a first camera assembly, a second camera assembly, a third camera assembly, and a fourth camera assembly;
the first camera assembly and the second camera assembly are configured for acquiring emission lights with two different wavelengths generated in the first region of the sample of interest;
the third camera assembly and the fourth camera assembly are configured for acquiring emission lights with another two wavelengths generated in the second region of the sample of interest.

13. The optomechanical system according to item 11 or 12, wherein

the beamsplitter assembly comprises a first dichroic mirror, a second dichroic mirror, a third dichroic mirror, a fourth dichroic mirror, and a fifth dichroic mirror;
the first dichroic mirror and the second dichroic mirror are separately arranged on the optical axis of the lens assembly, and the first dichroic mirror is configured for reflecting the excitation light to the lens assembly to project the excitation light to the sample of interest through the lens assembly;
the second dichroic mirror is configured for receiving the emission light transmitted by the first dichroic mirror and reflecting the emission light to the third dichroic mirror;
the third dichroic mirror is configured for receiving the emission light reflected by the second dichroic mirror, splitting the emission light into a first light beam with mixed wavelengths and a second light beam with mixed wavelengths, reflecting the first light beam with mixed wavelengths to the fourth dichroic mirror, and transmitting the second light beam with mixed wavelengths to the fifth dichroic mirror;
the first light beam with mixed wavelengths comprises a light beam with a first wavelength and a light beam with a second wavelength, and the second light beam with mixed wavelengths comprises a light beam with a third wavelength and a light beam with a fourth wavelength;
the fourth dichroic mirror is configured for receiving the first light beam with mixed wavelengths, transmitting the light beam with the first wavelength of the first light beam with mixed wavelengths to the first camera assembly, and reflecting the light beam with the second wavelength of the first light beam with mixed wavelengths to the second camera assembly;
the fifth dichroic mirror is configured for receiving the second light beam with mixed wavelengths, transmitting the light beam with the third wavelength of the second light beam with mixed wavelengths to the third camera assembly, and reflecting the light beam with the fourth wavelength of the second light beam with mixed wavelengths to the fourth camera assembly.

14. The optomechanical system according to any one of items 11-13, wherein

each of the camera assemblies comprises a tube lens and a TDI camera;
the tube lens is configured for converging the emission light to the TDI camera and for aberration correction, apochromatic imaging, and field curvature reduction.

15. The optomechanical system according to item 14, wherein

the tube lens comprises a first lens, a second lens, a third lens, a fourth lens and a fifth lens that are arranged in sequence from an object side to an image side;
the first lens has a negative refractive power;
the second lens has a positive refractive power;
the third lens has a positive refractive power;
the fourth lens has a positive refractive power;
the fifth lens has a negative refractive power;
object-side surfaces and image-side surfaces of the first lens, the second lens, the third lens, the fourth lens, and the fifth lens are all spherical surfaces.

16. The optomechanical system according to item 15, wherein

the object-side surface of the first lens is convex at the optical axis of the first lens, and the image-side surface of the first lens is concave at the optical axis of the first lens; the object-side surface of the first lens has a curvature radius of 100 mm to 200 mm at the optical axis of the first lens; and/or the image-side surface of the first lens has a curvature radius of 50 mm to 200 mm at the optical axis of the first lens.

17. The optomechanical system according to item 15 or 16, wherein

the object-side surface of the second lens is convex at the optical axis of the second lens, and the image-side surface of the second lens is convex at the optical axis of the second lens; the object-side surface of the second lens has a curvature radius of 50 mm to 200 mm at the optical axis of the second lens; and/or the image-side surface of the second lens has a curvature radius of -100 mm to -500 mm at the optical axis of the second lens.

18. The optomechanical system according to any one of items 15-17, wherein

the object-side surface of the third lens is convex at the optical axis of the third lens, and the image-side surface of the third lens is planar at the optical axis of the third lens; the object-side surface of the third lens has a curvature radius of 50 mm to 200 mm at the optical axis of the third lens; and/or the image-side surface of the third lens has a curvature radius of 100 mm to 500 mm at the optical axis of the third lens.

19. The optomechanical system according to any one of items 15-18, wherein

the object-side surface of the fourth lens is planar at the optical axis of the fourth lens, and the image-side surface of the fourth lens is convex at the optical axis of the fourth lens; the object-side surface of the fourth lens has a curvature radius greater than 1000 mm at the optical axis of the fourth lens; and/or the image-side surface of the fourth lens has a curvature radius of -100 mm to -500 mm at the optical axis of the fourth lens.

20. The optomechanical system according to any one of items 15-19, wherein

the object-side surface of the fifth lens is concave at the optical axis of the fifth lens, and the image-side surface of the fifth lens is concave at the optical axis of the fifth lens; the object-side surface of the fifth lens has a curvature radius of -100 mm to -500 mm at the optical axis of the fifth lens; and/or the image-side surface of the fifth lens has a curvature radius of 50 mm to -200 mm at the optical axis of the fifth lens.

21. The optomechanical system according to any one of items 15-20, wherein

the first lens has a thickness of 5 to 20 mm at the optical axis of the first lens; and/or, the second lens has a thickness of 5 to 20 mm at the optical axis of the second lens; and/or, the third lens has a thickness of 5 to 20 mm at the optical axis of the third lens; and/or, the fourth lens has a thickness of 5 to 20 mm at the optical axis of the fourth lens; and/or, the fifth lens has a thickness of 5 to 20 mm at the optical axis of the fifth lens.

22. The optomechanical system according to any one of items 15-21, wherein the first lens and the second lens are cemented to form a first cemented lens set; and/or, the fourth lens and the fifth lens are cemented to form a second cemented lens set.

23. The optomechanical system according to any one of items 15-22, wherein

the tube lens meets the relationship equation:

$$\frac{\Delta l'_{FC}}{\beta^2} \leq \frac{DOF}{5},$$

the $\Delta l'_{FC}$ is the axial chromatic aberration, the $\beta$ is the magnification, and the DOF is the depth of field.

24. The optomechanical system according to item 23, wherein the $\Delta l'_{FC}$ is less than or equal to 100 mm.

25. The optomechanical system according to any one of items 15-24, wherein the tube lens has a transmittance greater than 93%.

26. The optomechanical system according to any one of items 15-25, wherein each of the camera assemblies further comprises a filter arranged on one side of the first lens distal to the second lens.

27. The optomechanical system according to any one of items 13-26, wherein the imaging device further comprises an optical path turning assembly; the optical path turning assembly comprises a first base; the first dichroic mirror is adjustably connected to the first base; the illumination device is connected to the first base, and an excitation light output port of the illumination device is oriented towards the first dichroic mirror to reflect the excitation light to the lens assembly through the first dichroic mirror.

28. The optomechanical system according to item 27, wherein

the imaging device further comprises a first adjusting assembly; the first adjusting assembly comprises a first adjusting member and a first fixing member; the first adjusting member is in a threaded connection with the second dichroic mirror and abuts against the first base; the first fixing member is arranged through the second dichroic mirror and is connected to the first base.

29. The optomechanical system according to item 27 or 28, wherein

the optical path turning assembly further comprises a first positioning insert block; the first positioning insert block is detachably connected to the first base and is arranged on the side towards the second dichroic mirror; the first adjusting member abuts against the first positioning insert block.

30. The optomechanical system according to any one of items 27-29, wherein the second dichroic mirror is connected to the first base and arranged downstream on the optical path of the first dichroic mirror along the optical axis of the lens assembly.

31. The optomechanical system according to item 30, wherein

the optomechanical system further comprises an automatic focusing device, and the automatic focusing device is connected to the first base; the automatic focusing device is configured for emitting a focusing light beam, such that the focusing light beam sequentially passes through the second dichroic mirror, the first dichroic mirror, and the lens assembly, and then irradiates the sample of interest; the automatic focusing device is further configured for receiving the focusing light beam reflected by the sample of interest.

32. The optomechanical system according to item 31, wherein

the automatic focusing device comprises a second base, a focusing assembly, and a second adjusting assembly; the second base is connected to the first base, and the focusing assembly is connected to the second base; the second adjusting assembly is configured for adjusting relative positions between the focusing assembly and the second base.

33. The optomechanical system according to item 32, wherein

the second adjusting assembly comprises a first movable seat and a second adjusting member; the first movable seat is movably arranged relative to the second base; the focusing assembly is arranged on the first movable seat; the second adjusting member abuts against the second base and is connected to the first movable seat; when the second adjusting member is operated, the pitch angle of the focusing assembly relative to the second base is adjusted.

34. The optomechanical system according to item 32 or 33, wherein

the second adjusting assembly comprises a second movable seat and a third adjusting member; the second movable seat is movably arranged relative to the second base; the focusing assembly is arranged on the second movable seat; the third adjusting member is separately connected to the second movable seat and the second base; when the third adjusting member is operated, the second movable seat moves relative to the second base.

35. The optomechanical system according to any one of items 11-34, wherein

the lens assembly comprises an objective lens structure and a third base; the objective lens structure is connected to the third base and arranged between the sample of interest and the first dichroic mirror.

36. The optomechanical system according to item 35, wherein the objective lens structure comprises an objective lens and a mounting bracket, the mounting bracket and the third base are movable relative to each other, and the objective lens is detachably connected to the mounting bracket.

37. The optomechanical system according to item 36, wherein

the objective lens is configured for projecting the excitation light to the sample of interest and acquiring the emission light generated by the sample of interest, and has a numerical aperture greater than 0.75 and a field of view greater than 1.2 mm.

38. The optomechanical system according to item 36 or 37, wherein

the objective lens is provided with a first light-transmitting region and a second light-transmitting region;
the first light-transmitting region is configured for projecting the excitation light emitted by the first light source into the first region of the sample of interest and receiving the emission light generated in the first region;
the second light-transmitting region is configured for projecting the excitation light emitted by the second light source into the second region of the sample of interest and receiving the emission light generated in the second region.

39. The optomechanical system according to any one of items 35-38, wherein
the lens assembly further comprises a lifting structure; the lifting structure is separately connected to the third base and the objective lens structure and configured for driving the objective lens structure to move up and down relative to the third base.

40. The optomechanical system according to item 39, wherein
the lifting structure comprises a driving member and a stopper; the stopper is connected to the objective structure, and is at least partially positioned on the top of the third base; the driving member is separately connected to the objective lens structure and the third base and configured for driving the objective lens structure to move.

41. The optomechanical system according to item 39 or 40, wherein
the lens assembly comprises a first spring; the first spring is arranged on the lifting structure and configured for applying an elastic force to the objective lens structure when the lifting structure drives the objective lens structure to move.

42. The optomechanical system according to any one of items 35-41, wherein
the optomechanical system further comprises a bracket device; the lens assembly further comprises a third adjusting assembly; the third adjusting assembly is connected to the third base, abuts against the bracket device, and is configured for adjusting the third base to move relative to the bracket device.

43. The optomechanical system according to item 42, wherein
the third adjusting assembly comprises a second positioning insert block and a fourth adjusting member; the second positioning insert block is detachably connected to the bracket device; the fourth adjusting member is in a threaded connection with the third base, and abuts against the second positioning insert block.

44. The optomechanical system according to item 42 or 43, wherein
the lens assembly further comprises a third fixing member and a second spring; the third fixing member is separately connected to the third base and the bracket device; the second spring is arranged through the third base and connected to the bracket device to drive the third base to have a tendency of moving towards the bracket device.

45. The optomechanical system according to any one of items 1-44, wherein
the illumination device comprises an illumination assembly and a fourth adjusting assembly; the fourth adjusting assembly comprises at least one of a linear adjusting mechanism, a rotational adjusting mechanism, and a pitch adjusting mechanism, and is connected to the illumination assembly and configured for driving the illumination assembly to move relative to the imaging device.

46. The optomechanical system according to any one of items 1-45, wherein

the optomechanical system further comprises the bracket device, and a cavity is arranged in the bracket device;
the movable platform device comprises a carrier structure and a movable carrier platform; the carrier structure is provided with a receiving slot configured for receiving the sample of interest; the carrier structure is arranged on the movable carrier platform; the movable carrier platform is connected to the bracket device, arranged in the cavity, and configured for driving the carrier structure to conduct at least one of linear motion and rotary motion relative to the imaging device.

47. The optomechanical system according to item 46, wherein
the carrier structure comprises a carrier stage and a locking member; the locking member is movably connected to the carrier stage; the receiving slot is arranged on the carrier stage; the sample of interest is provided with a positioning slot; the locking member is configured for snap-fitting with the positioning slot to fix the sample of interest in the receiving slot.

48. The optomechanical system according to item 46 or 47, wherein
the carrier structure comprises the carrier stage and a temperature controller; the receiving slot is arranged on the carrier stage; the temperature controller comprises a heating stage and a temperature sensor; the heating stage is arranged at the bottom of the receiving slot; the temperature sensor is connected to the carrier stage and configured for acquiring the temperature of the sample of interest.

49. The optomechanical system according to any one of items 46-48, wherein
the movable carrier platform comprises a rotary stage and a movable carrier stage; the rotary stage is separately connected to the movable carrier stage and the carrier structure, and configured for driving the carrier structure to rotate relative to the imaging device; the movable carrier stage is configured for driving the carrier structure to move along at least one direction relative to the imaging device.

50. The optomechanical system according to any one of items 1-49, wherein
the optomechanical system further comprises the bracket device; a cavity configured for receiving the movable platform device is arranged in the bracket device; a mounting surface is arranged at the top of the bracket device; the illumination device and the imaging device are arranged on the mounting surface.

51. The optomechanical system according to any one of items 1-50, wherein
the bracket device comprises a frame, a vibration damping structure, and a bottom plate structure; the frame is arranged apart from the bottom plate structure and is positioned over the bottom plate structure; the vibration damping structure is separately connected to the frame and the bottom plate structure.

52. The optomechanical system according to item 51, wherein
the frame is provided with an accommodation hole in separate communication with the cavity and the mounting surface; the lens assembly is accommodated in the accommodation hole.

53. A sequencing system, comprising the optomechanical system according to any one of items 1-52.

**Claims**

1. An optomechanical system, comprising:

    an illumination device, configured for emitting an excitation light to irradiate a sample of interest so as to excite the sample of interest to generate an emission light;
    an imaging device, configured for acquiring the emission light and forming an image; and
    a movable platform device, configured for driving the sample of interest to translate and/or rotate relative to the imaging device, so as to enable the imaging device to consecutively acquire the emission light.

2. The optomechanical system according to claim 1, wherein

    the illumination device comprises one or more light sources, and the light sources are configured for emitting a plurality of excitation lights with different wavelengths;
    the plurality of excitation lights with different wavelengths respectively irradiate different regions of the sample of interest along different illumination optical paths;
    optionally, the illumination device comprises a shaping lens set;
    the shaping lens set is positioned on an illumination optical path of the excitation light;
    the shaping lens set is configured for shaping the excitation light in a first direction and a second direction perpendicular to the first direction to adapt a shape and/or size of a spot of the excitation light to an imaging region of the imaging device;
    optionally, the illumination device comprises a collimation lens;
    the collimation lens is positioned on an upstream or downstream optical path of the shaping lens set.

3. The optomechanical system according to claim 2, wherein
the light sources comprise a first light source and a second light source; the first light source and the second light source are respectively configured for emitting an excitation light with a first wavelength and an excitation light with a second wavelength; the excitation light with the first wavelength irradiates a first region of the sample of interest along a first illumination optical path to form a first spot, and the excitation light with the second wavelength irradiates the second region of the sample of interest along a second illumination optical path to form a second spot; the first spot does not overlap with the second spot.

4. The optomechanical system according to claim 3, wherein

    the shaping lens set comprises a first shaping lens set and a second shaping lens set;
    the first shaping lens set is arranged on the first illumination optical path, and the excitation light with the first wavelength irradiates the first region of the sample of interest after being shaped by the first shaping lens set to form an elongated first spot;
    the second shaping lens set is arranged on the second illumination optical path, and the excitation light emitted by the second light source irradiates the second region of the sample of interest after being shaped by the second shaping lens set to form an elongated second spot;
    optionally, the first shaping lens set and the second shaping lens set each comprise a first cylindrical lens and a second cylindrical lens; the first cylindrical lens and the second cylindrical lens are respectively configured for zooming the excitation light in the first direction and the second direction to adapt the shape and/or size of the spot of the excitation light to the imaging region of the imaging device.

5. The optomechanical system according to claim 4, wherein

    the collimation lens comprises a first collimation lens and a second collimation lens;
    the first collimation lens is positioned on an upstream or downstream optical path of the first shaping lens set;
    the second collimation lens is positioned on an upstream or downstream optical path of the second shaping lens set; optionally, end faces of the illumination device for outputting the excitation light of the first wavelength and the excitation light of the second wavelength are rectangular.

**6.** The optomechanical system according to any one of claims 1-5, wherein

the imaging device comprises a lens assembly, a beamsplitter assembly, and a plurality of camera assemblies;

the lens assembly is configured for projecting the excitation light to the sample of interest, acquiring the emission light generated by the sample of interest, and projecting the emission light to the beamsplitter assembly;

the beamsplitter assembly is configured for receiving the emission light acquired by the lens assembly, splitting the emission light, and projecting the emission light into the plurality of camera assemblies;

the plurality of camera assemblies are configured for acquiring the emission light and forming the image.

**7.** The optomechanical system according to claim 6, wherein

the plurality of camera assemblies comprises four camera assemblies, i.e., a first camera assembly, a second camera assembly, a third camera assembly, and a fourth camera assembly;

the first camera assembly and the second camera assembly are configured for acquiring emission lights with two different wavelengths generated in the first region of the sample of interest;

the third camera assembly and the fourth camera assembly are configured for acquiring emission lights with another two wavelengths generated in the second region of the sample of interest.

**8.** The optomechanical system according to claim 6 or 7, wherein

the beamsplitter assembly comprises a first dichroic mirror, a second dichroic mirror, a third dichroic mirror, a fourth dichroic mirror, and a fifth dichroic mirror;

the first dichroic mirror and the second dichroic mirror are separately arranged on the optical axis of the lens assembly, and the first dichroic mirror is configured for reflecting the excitation light to the lens assembly to project the excitation light to the sample of interest through the lens assembly;

the second dichroic mirror is configured for receiving the emission light transmitted by the first dichroic mirror and reflecting the emission light to the third dichroic mirror;

the third dichroic mirror is configured for receiving the emission light reflected by the second dichroic mirror, splitting the emission light into a first light beam with mixed wavelengths and a second light beam with mixed wavelengths, reflecting the first light beam with mixed wavelengths to the fourth dichroic mirror, and transmitting the second light beam with mixed wavelengths to the fifth dichroic mirror;

the first light beam with mixed wavelengths comprises a light beam with a first wavelength and a light beam with a second wavelength, and the second light beam with mixed wavelengths comprises a light beam with a third wavelength and a light beam with a fourth wavelength;

the fourth dichroic mirror is configured for receiving the first light beam with mixed wavelengths, transmitting the light beam with the first wavelength of the first light beam with mixed wavelengths to the first camera assembly, and reflecting the light beam with the second wavelength of the first light beam with mixed wavelengths to the second camera assembly;

the fifth dichroic mirror is configured for receiving the second light beam with mixed wavelengths, transmitting the light beam with the third wavelength of the second light beam with mixed wavelengths to the third camera assembly, and reflecting the light beam with the fourth wavelength of the second light beam with mixed wavelengths to the fourth camera assembly;

optionally, each of the camera assemblies comprises a tube lens and a TDI camera;

the tube lens is configured for converging the emission light to the TDI camera and for aberration correction, apochromatic imaging, and field curvature reduction;

optionally, the tube lens comprises a first lens, a second lens, a third lens, a fourth lens and a fifth lens that are arranged in sequence from an object side to an image side;

the first lens has a negative refractive power;
the second lens has a positive refractive power;
the third lens has a positive refractive power;
the fourth lens has a positive refractive power;
the fifth lens has a negative refractive power;

object-side surfaces and image-side surfaces of the first lens, the second lens, the third lens, the fourth lens, and the fifth lens are all spherical surfaces;

optionally, the object-side surface of the first lens is convex at the optical axis of the first lens, and the image-side surface of the first lens is concave at the optical axis of the first lens;

the object-side surface of the first lens has a curvature radius of 100 mm to 200 mm at the optical axis of the first lens; and/or the image-side surface of the first lens has a curvature radius of 50 mm to 200 mm at the optical axis of the first lens;

optionally, the object-side surface of the second lens is convex at the optical axis of the second lens, and the image-side surface of the second lens is convex at the optical axis of the second lens;

the object-side surface of the second lens has a curvature radius of 50 mm to 200 mm at the optical axis of the second lens; and/or the image-side surface of the second lens has a curvature radius of -100 mm to -500 mm at the optical axis of the second lens;

optionally, the object-side surface of the third lens is convex at the optical axis of the third lens, and the image-side surface of the third lens is planar at the optical axis of the third lens;

the object-side surface of the third lens has a curvature radius of 50 mm to 200 mm at the optical axis of the third lens; and/or the image-side surface of the third lens has a curvature radius of 100 mm to 500 mm at the optical axis of the third lens;

optionally, the object-side surface of the fourth lens is planar at the optical axis of the fourth lens, and the image-side surface of the fourth lens is convex at the optical axis of the fourth lens;

the object-side surface of the fourth lens has a curvature radius greater than 1000 mm at the optical axis of the fourth lens; and/or the image-side surface of the fourth lens has a curvature radius of -100 mm to -500 mm at the optical axis of the fourth lens;

optionally, the object-side surface of the fifth lens is concave at the optical axis of the fifth lens, and the image-side surface of the fifth lens is concave at the optical axis of the fifth lens;

the object-side surface of the fifth lens has a curvature radius of -100 mm to -500 mm at the optical axis of the fifth lens; and/or the image-side surface of the fifth lens has a curvature radius of 50 mm to -200 mm at the optical axis of the fifth lens;

optionally, the first lens has a thickness of 5 to 20 mm at the optical axis of the first lens; and/or, the second lens has a thickness of 5 to 20 mm at the optical axis of the second lens; and/or, the third lens has a thickness of 5 to 20 mm at the optical axis of the third lens; and/or, the fourth lens has a thickness of 5 to 20 mm at the optical axis of the fourth lens; and/or, the fifth lens has a thickness of 5 to 20 mm at the optical axis of the fifth lens;

optionally, the first lens and the second lens are cemented to form a first cemented lens set; and/or, the fourth lens and the fifth lens are cemented to form a second cemented lens set;

optionally, the tube lens meets the relationship

equation: $\frac{\Delta l'_{FC}}{\beta^2} \leq \frac{DOF}{5}$ .

the $\Delta l'_{FC}$ is the axial chromatic aberration, the $\beta$ is the magnification, and the DOF is the depth of field; optionally, the $\Delta l'_{FC}$ is less than or equal to 100 mm;

optionally, the tube lens has a transmittance greater than 93%;

optionally, each of the camera assemblies further comprises a filter arranged on one side of the first lens distal to the second lens.

9. The optomechanical system according to claim 8, wherein

the imaging device further comprises an optical path turning assembly; the optical path turning assembly comprises a first base; the first dichroic mirror is adjustably connected to the first base; the illumination device is connected to the first base, and an excitation light output port of the illumination device is oriented towards the first dichroic mirror to reflect the excitation light to the lens assembly through the first dichroic mirror;

the imaging device further comprises a first adjusting assembly; the first adjusting assembly comprises a first adjusting member and a first fixing member; the first adjusting member is in a threaded connection with the second dichroic mirror and abuts against the first base; the first fixing member is arranged through the second dichroic mirror and is connected to the first base; and/or

the optical path turning assembly further comprises a first positioning insert block; the first positioning insert block is detachably connected to the first base and is arranged on the side towards the second dichroic mirror; the first adjusting member abuts against the first positioning insert block;

optionally, the second dichroic mirror is connected to the first base and arranged downstream on the optical path of the first dichroic mirror along the optical axis of the lens assembly.

10. The optomechanical system according to any one of claims 1-9, wherein

the optomechanical system further comprises an automatic focusing device, and the automatic focusing device is connected to the first base; the automatic focusing device is configured for emitting a focusing light beam, such that the focusing light beam sequentially passes through the second dichroic mirror, the first dichroic mirror, and the lens assembly, and then irradiates the sample of interest; the automatic focusing device is further configured for receiving the focusing light beam reflected by the sample of interest.

11. The optomechanical system according to claim 10, wherein

the automatic focusing device comprises a second base, a focusing assembly, and a second adjusting assembly; the second base is connected to the first base, and the focusing assembly is connected to the second base; the second adjusting assembly comprises a first movable seat and a second adjusting member; the first movable seat is movably arranged relative to the second base; the focusing assembly is arranged on the first movable seat; the second adjusting member abuts against the second base and is connected to the first movable seat; when the second adjusting member is operated, the pitch angle of the focusing assembly relative to the second base is adjusted.

12. The optomechanical system according to claim 11, wherein
the second adjusting assembly comprises a second movable seat and a third adjusting member; the second movable seat is movably arranged relative to the second base; the focusing assembly is arranged on the second movable seat; the third adjusting member is separately connected to the second movable seat and the second base; when the third adjusting member is operated, the second movable seat moves relative to the second base.

13. The optomechanical system according to any one of claims 6-12, wherein

the lens assembly comprises an objective lens structure and a third base; the objective lens structure is connected to the third base and arranged between the sample of interest and the first dichroic mirror; the objective lens structure comprises an objective lens and a mounting bracket, the mounting bracket and the third base are movable relative to each other, and the objective lens is detachably connected to the mounting bracket; the objective lens is configured for projecting the excitation light to the sample of interest and acquiring the emission light generated by the sample of interest, and has a numerical aperture greater than 0.75 and a field of view greater than 1.2 mm.

14. The optomechanical system according to claim 13, wherein

the objective lens is provided with a first light-transmitting region and a second light-transmitting region;
the first light-transmitting region is configured for projecting the excitation light emitted by the first light source into the first region of the sample of interest and receiving the emission light generated in the first region;
the second light-transmitting region is configured for projecting the excitation light emitted by the second light source into the second region of the sample of interest and receiving the emission light generated in the second region.
optionally, the lens assembly further comprises a lifting structure; the lifting structure is separately connected to the third base and the objective lens structure and configured for driving the objective lens structure to move up and down relative to the third base;
optionally, the lifting structure comprises a driving member and a stopper; the stopper is connected to the objective structure, and is at least partially positioned on the top of the third base; the driving member is separately connected to the objective lens structure and the third base and configured for driving the objective lens structure to move; optionally, the lens assembly comprises a first spring; the first spring is arranged on the lifting structure and configured for applying an elastic force to the objective lens structure when the lifting structure drives the objective lens structure to move;
optionally, the optomechanical system further comprises a bracket device; the lens assembly further comprises a third adjusting assembly; the third adjusting assembly is connected to the third base, abuts against the bracket device, and is configured for adjusting the third base to move relative to the bracket device;
optionally, the third adjusting assembly comprises a second positioning insert block and a fourth adjusting member; the second positioning insert block is detachably connected to the bracket device; the fourth adjusting member is in a threaded connection with the third base, and abuts against the second positioning insert block;
optionally, the lens assembly further comprises a third fixing member and a second spring; the third fixing member is separately connected to the third base and the bracket device; the second spring is arranged through the third base and connected to the bracket device to drive the third base to have a tendency of moving towards the bracket device; optionally, the illumination device comprises an illumination assembly and a fourth adjusting assembly; the fourth adjusting assembly comprises at least one of a linear adjusting mechanism, a rotational adjusting mechanism, and a pitch adjusting mechanism, and is connected to the illumination assembly and configured for driving the illumination assembly to move relative to the imaging device; optionally, the optomechanical system further comprises the bracket device, and a cavity is arranged in the bracket device; the movable platform device comprises a carrier structure and a movable carrier platform; the carrier structure is provided with a receiving slot configured for receiving the sample of interest; the carrier structure is arranged on the movable carrier platform; the movable carrier platform is connected to the bracket device, arranged in the cavity, and configured for driving the carrier structure to conduct at least one of linear motion and rotatory motion relative to the imaging device;

optionally, the carrier structure comprises a carrier stage and a locking member; the locking member is movably connected to the carrier stage; the receiving slot is arranged on the carrier stage; the sample of interest is provided with a positioning slot; the locking member is configured for snap-fitting with the positioning slot to fix the sample of interest in the receiving slot; optionally, the carrier structure comprises the carrier stage and a temperature controller; the receiving slot is arranged on the carrier stage; the temperature controller comprises a heating stage and a temperature sensor; the heating stage is arranged at the bottom of the receiving slot; the temperature sensor is connected to the carrier stage and configured for acquiring the temperature of the sample of interest; optionally, the movable carrier platform comprises a rotary stage and a movable carrier stage; the rotary stage is separately connected to the movable carrier stage and the carrier structure, and configured for driving the carrier structure to rotate relative to the imaging device; the movable carrier stage is configured for driving the carrier structure to move along at least one direction relative to the imaging device; optionally, the optomechanical system further comprises the bracket device; a cavity configured for receiving the movable platform device is arranged in the bracket device; a mounting surface is arranged at the top of the bracket device; the illumination device and the imaging device are arranged on the mounting surface; optionally, the bracket device comprises a frame, a vibration damping structure, and a bottom plate structure; the frame is arranged apart from the bottom plate structure and is positioned over the bottom plate structure; the vibration damping structure is separately connected to the frame and the bottom plate structure; optionally, the frame is provided with an accommodation hole in separate communication with the cavity and the mounting surface; the lens assembly is accommodated in the accommodation hole.

15. A sequencing system, comprising the optomechanical system according to any one of claims 1-14.

10

FIG. 1

FIG. 2

FIG. 3

FIG. 4

FIG. 5

FIG. 6

FIG. 7

FIG. 8

A

FIG. 9

FIG. 10

FIG. 11

FIG. 12

FIG. 13

FIG. 14

FIG. 15

Surface: IMA

| Spot Diagram | | | | |
|---|---|---|---|---|
| LENS HAS NO TITLE., DEC. 7, 2022 | | | | Zemax |
| Units are μm. Airy radius: 8.683 μm. Legend items refer to Wavelengths | | | | Zemax OpticStudio 18.9 |
| Field: | 1 | 2 | 3 | 4 |
| RMS radius: | 1.318 | 3.745 | 4.558 | 4.041 |
| GEO radius: | 2.261 | 8.645 | 12.036 | 9.542 | GiantTube-+_ZMX |
| Scale bar: | 40 | Reference: | Chief Ray | Configuration 1 of 2 |

FIG. 16

## EUROPEAN SEARCH REPORT

Application Number

EP 24 21 6343

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/077306 A1 (MGI TECH CO LTD [CN]) 11 May 2023 (2023-05-11) | 1-7, 13-15 | INV. G01N21/64 C12Q1/6869 |
| Y | * page 17 - page 18; figures 1-4 * & US 2025/059599 A1 (WEI YI [CN] ET AL) 20 February 2025 (2025-02-20) * paragraphs [0001], [0067] - [0074]; figures 1-4 * | 8-12 | |
| X | US 2008/117425 A1 (KAIN ROBERT [US]) 22 May 2008 (2008-05-22) | 1-6, 13-15 | |
| Y | * paragraphs [0003], [0043] - [0053], [0059], [0080], [0082] - [0085]; figures 1-5,14 * | 9-12 | |
| X | EP 3 373 062 A1 (ILLUMINA INC [US]) 12 September 2018 (2018-09-12) | 1,15 | |
| Y | * paragraphs [0018], [0038] - [0040], [0047], [0051], [0058] - [0063], [0082] - [0083]; figure 1 * | 8-12 | |
| X | US 2023/092006 A1 (JIANG HEMING [CN] ET AL) 23 March 2023 (2023-03-23) | 1,6,7, 13-15 | **TECHNICAL FIELDS SEARCHED (IPC)** |
| Y | * paragraphs [0035] - [0039]; figure 1 * | 8-12 | G01N C12Q |
| Y | US 2022/251644 A1 (CHEN STEVE XIANGLING [US] ET AL) 11 August 2022 (2022-08-11) * figure 18 * | 8-12 | |

The present search report has been drawn up for all claims

| Place of search | Date of completion of the search | Examiner |
|---|---|---|
| Munich | 3 April 2025 | Riblet, Philippe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 21 6343

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2025

| Patent document cited in search report | | Publication date | Patent family member(s) | | Publication date |
|---|---|---|---|---|---|
| WO 2023077306 | A1 | 11-05-2023 | AU 2021472582 A1 | | 16-05-2024 |
| | | | CA 3236241 A1 | | 11-05-2023 |
| | | | CN 117716017 A | | 15-03-2024 |
| | | | EP 4394027 A1 | | 03-07-2024 |
| | | | JP 2024539759 A | | 30-10-2024 |
| | | | US 2025059599 A1 | | 20-02-2025 |
| | | | WO 2023077306 A1 | | 11-05-2023 |
| US 2008117425 | A1 | 22-05-2008 | US 2008117425 A1 | | 22-05-2008 |
| | | | US 2010328732 A1 | | 30-12-2010 |
| EP 3373062 | A1 | 12-09-2018 | AU 2018201388 A1 | | 27-09-2018 |
| | | | BR 102018004635 A2 | | 30-10-2018 |
| | | | CA 2996788 A1 | | 08-09-2018 |
| | | | CN 108572439 A | | 25-09-2018 |
| | | | CN 208705562 U | | 05-04-2019 |
| | | | EP 3373062 A1 | | 12-09-2018 |
| | | | IL 257830 A | | 30-04-2018 |
| | | | JP 6782268 B2 | | 11-11-2020 |
| | | | JP 2018151624 A | | 27-09-2018 |
| | | | JP 2020170165 A | | 15-10-2020 |
| | | | KR 20180103014 A | | 18-09-2018 |
| | | | KR 20200003945 A | | 10-01-2020 |
| | | | MY 196535 A | | 19-04-2023 |
| | | | NZ 740257 A | | 30-08-2019 |
| | | | SG 10201801707X A | | 30-10-2018 |
| | | | TW 201843491 A | | 16-12-2018 |
| | | | US 2018258468 A1 | | 13-09-2018 |
| US 2023092006 | A1 | 23-03-2023 | CN 115380203 A | | 22-11-2022 |
| | | | EP 4105642 A1 | | 21-12-2022 |
| | | | JP 7511013 B2 | | 04-07-2024 |
| | | | JP 2023513729 A | | 03-04-2023 |
| | | | US 2023092006 A1 | | 23-03-2023 |
| | | | WO 2021159285 A1 | | 19-08-2021 |
| US 2022251644 | A1 | 11-08-2022 | AU 2021207987 A1 | | 18-11-2021 |
| | | | AU 2021254575 A1 | | 18-11-2021 |
| | | | AU 2022204320 A1 | | 07-07-2022 |
| | | | AU 2024205076 A1 | | 15-08-2024 |
| | | | CA 3137050 A1 | | 22-07-2021 |
| | | | CN 113840924 A | | 24-12-2021 |
| | | | CN 114410761 A | | 29-04-2022 |
| | | | CN 115261381 A | | 01-11-2022 |
| | | | CN 115287341 A | | 04-11-2022 |
| | | | CN 115369157 A | | 22-11-2022 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 24 21 6343

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2025

| Patent document cited in search report | Publication date | Patent family member(s) | Publication date |
|---|---|---|---|
| | | DE 112021000050 T5 | 25-05-2022 |
| | | DE 202021001858 U1 | 05-08-2021 |
| | | EP 3942039 A1 | 26-01-2022 |
| | | EP 3988922 A1 | 27-04-2022 |
| | | EP 4056988 A1 | 14-09-2022 |
| | | EP 4060321 A1 | 21-09-2022 |
| | | EP 4089182 A1 | 16-11-2022 |
| | | GB 2598233 A | 23-02-2022 |
| | | GB 2598498 A | 02-03-2022 |
| | | GB 2604072 A | 24-08-2022 |
| | | GB 2604297 A | 31-08-2022 |
| | | GB 2605310 A | 28-09-2022 |
| | | GB 2615914 A | 23-08-2023 |
| | | GB 2616362 A | 06-09-2023 |
| | | GB 2626884 A | 07-08-2024 |
| | | IL 287375 A | 01-12-2021 |
| | | IL 287430 A | 01-12-2021 |
| | | IL 303164 A | 01-07-2023 |
| | | IL 311540 A | 01-05-2024 |
| | | JP 7223165 B2 | 15-02-2023 |
| | | JP 2022129394 A | 05-09-2022 |
| | | JP 2022540970 A | 21-09-2022 |
| | | KR 20210154868 A | 21-12-2021 |
| | | KR 20220118300 A | 25-08-2022 |
| | | SG 10202112013Q A | 30-12-2021 |
| | | SG 11202111573V A | 29-11-2021 |
| | | US 11053540 B1 | 06-07-2021 |
| | | US 11060138 B1 | 13-07-2021 |
| | | US 2021223161 A1 | 22-07-2021 |
| | | US 2021332416 A1 | 28-10-2021 |
| | | US 2021333210 A1 | 28-10-2021 |
| | | US 2021333211 A1 | 28-10-2021 |
| | | US 2022025457 A1 | 27-01-2022 |
| | | US 2022136047 A1 | 05-05-2022 |
| | | US 2022251643 A1 | 11-08-2022 |
| | | US 2022251644 A1 | 11-08-2022 |
| | | US 2022267842 A1 | 25-08-2022 |
| | | WO 2021146597 A1 | 22-07-2021 |

EPO FORM P0459

For more details about this annex : see Official Journal of the European Patent Office, No. 12/82

page 2 of 2